# EUROPEAN PATENT SPECIFICATION

(11) **EP 0 454 494 B1**
(45) Date of publication and mention of the grant of the patent: **20.09.1995**
(21) Application number: 91303818.8
(22) Date of filing: 26.04.1991
(51) Int. Cl.: C07C 49/743, C07C 49/747, C07C 323/41, C07C 233/32, C07D 493/22, C07D 493/10, C07D 317/72

(54) **Calicheamicinone, derivatives and analogs thereof and methods of making the same**
Calicheamicinon, dessen Abkömmlinge und Analogen und Verfahren zu deren Herstellung
Calicheamicinone, ses dérivés et analogues et procédés pour leur fabrication

(30) Priority: 27.04.1990 US 515209
(43) Date of publication of application: 30.10.1991
(73) Proprietor: YALE UNIVERSITY, New Haven, CT 06516 (US)
(72) Inventor: Danishefsky, Samuel J., New Haven, Connecticut 06515 (US); Haseltine, John School of Chemistry, Atlanta, Georgia 30332 (US); Cabal, Maria Paz, Buffalo, New York 14214 (US); Iwasawa, Nobuharu, Hamden, Connecticut 06517 (US); Coleman, Robert S., Columbia, Carolina 29210 (US)
(74) Representative: Fisher, Adrian John

(56) References cited:
- EP-A- 0 289 030
- EP-A- 0 289 030
- GB-A- 2 179 649
- JOURNAL OF THE AMERICAN CHEMICAL SOCIETY. vol. 109, no. 11, 1987, Gaston, PA, US, pages 3461 - 3462, J. GOLIK ET AL
- JOURNAL OF THE AMERICAN CHEMICAL SOCIETY. vol. 109, no. 11, 1987, Gaston, PA, US, pages 3462 - 3464. J. GOLIK ET AL
- JOURNAL OF THE AMERICAN CHEMICAL SOCIETY. vol. 109, no. 11, 1987, Gaston, PA, US, pages 3464 - 3466, M.D. LEE ET AL
- JOURNAL OF THE AMERICAN CHEMICAL SOCIETY. vol. 109, no. 11, 1987, Gaston, PA, US, pages 3466 - 3468, M.D. LEE ET AL
- TETRAHEDRON LETTERS vol. 30, no. 29, 1989, pages 3765 - 3768 F.J. SCHOENEN

## Description

The present invention relates to the family of compounds that exhibit DNA cleaving, antibiotic and anti-tumor activities known as calicheamicins, and more particularly to the aglycone portion of a calicheamicin molecule known as calicheamicinone and to derivatives and analogs of calicheamicinone.

### Background

Recently there has been discovered a growing collection of antibiotics bearing novel patterns of interactive unsaturation. The antimicrobial and antitumor properties of these compounds [(a) For structure of calicheamicin γ₁ see: Lee et al., J. Am. Chem. Soc., 109:3464 (1987); Lee et al., J. Am. Chem. Soc., 109:3466 (1987); (b) for structure of esperamicin A₁, A₂, A_{1b} and X, see: Golik, J. et al., J. Am. Chem. Soc.; 109:3461 (1987); Golik et al., J. Am. Chem. Soc., 109:3462 (1987); Konishi et al., J. Antibiotics 1605 (1985); (c) for recent structural and biological studies of dynemicin A, see: Konishi et al., J. Antibiotics 1449 (1989); (d) for structural and synthetic studies in the neocarzinostatin area, see: Napier et al., Biochem. Biophys. Res. Comm. 89:635 (1979); AlbersSchönberg et al., Biochem. Biophys. Res. Comm. 95:1351 (1980); Koide et al., J. Antibiotics 33:342 (1980); Edo et al., Tetrahedron Lett. 331 (1985); Wender et al., Tetrahedron Lett. 29:909 (1988)] follow from their capacity to cut double stranded DNA [(a) Zein et al. Science, 240:1198 (1988); (b) Long et al., Proc. Natl. Acad. Sci. U.S.A., 86:2 (1989)].

Evidence has been accumulated that the effector species for DNA degradation in vitro are diyls arising from chemically induced Bergman type [(a) for the conversion of diethynyl olefins into 1,4-dehychlorobenzene biradicals under thermal conditions, see: Bergman, Acc Chem. Res.. 6:25 (1973); Jones et al., J. Am. Chem. Soc., 94:660 (1972); Lockhart et al., J. Am. Chem. Soc., 103:4082 (1981); Lockhart et al., J. Am. Chem. Soc., 103:4091 (1981); (b) for earlier explorations in this area, see: Darby et al., Chem. Commun., 1516 (1971)], bond reorganizations [(a) for the first demonstration that the prototype diynenes relating to calicheamicins and esperamicins could be converted into benzenes, see: Magnus et al., Am. Chem. Soc., 110:1626 (1988); Magnus et al., J. Am. Chem Soc., 110:6921 (1988); (b) for demonstrations of structural parameters for cyclization of novel cyclic conjugated enediynes, see: Nicolaou et al., J. Am. Chem. Soc., 110:4866 (1988); (c) for the modeling of the nucleophilic activation of neocarzinostatin, see: Myers, Tetrahedron Lett., 4493 (1987); Myers et al., J. Am. Chem. Soc., 110:7212 (1988); Hensens et al., J. Antibiotics, 761 (1989); Nagata et al., Tetrahedron Lett., 30:4995 (1989)] of the unsaturated loci.

In a suitable setting, such species have a proclivity for abstracting carbon-bound hydrogen atoms from deoxyribose units of oligonucleotides [Zein et al., J. Am. Chem. Soc., 111:6888 (1989)]. In some instances, the drug identifies sites for DNA degradation with remarkable sequence specificity [(a) Zein et al., Science, 244:697 (1989); (b) Hawley et al., Proc. Natl. Acad. Sci. U.S.A., 86:1105 (1989)]. The high in vitro potency of these compounds, their structural novelty, and their interesting mechanism of action have served to stimulate a large multidisciplinary effort addressed to their biology and chemistry. The eventual goal is that of developing cytotoxic agents which can be specifically directed to transformed, or otherwise diseased cells [Hamann et al., 197^{th} A.C.S. National Meeting, Division of Medicinal Chemistry, Dallas, Texas, abstract #71A (1989)].

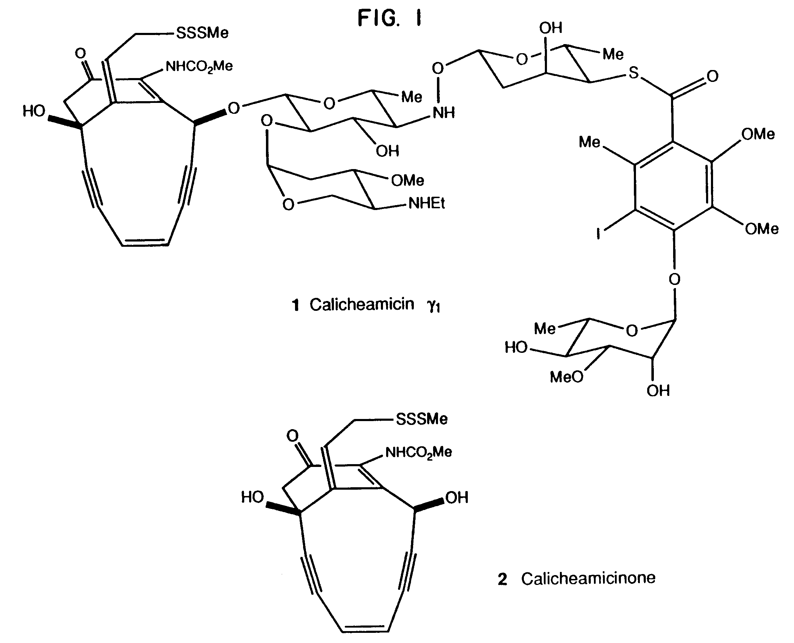

A fascinating example of such a drug is calicheamicin (Compound **1**, Figure 1) [(a) for structure of calicheamicin γ₁ see: Lee et al., J. Am. Chem. Soc., 109:3464 (1987); Lee et al., J. Am. Chem. Soc., 109:3466 (1987)]. The aglycone moiety calicheamicinone (Compound **2**, Figure 1), with its poised enediyne linkage, is perceived as the source of latent chemical radiation [Nicolaou et al, J. Am. Chem. Soc., 110:7247 (1988)]. The carbohydrate sector is seen to be the oligonucleotide recognition device [Hawley et al., Proc. Natl. Acad. Sci. U.S.A., 86:1105 (1989)].

It would therefore be of great interest to study these functions independently. However, at the present time, there have been no reports of disengagement of the intact carbocyclic and carbohydrate sectors of calicheamicin (or esperamicin, a drug having an aglycone portion similar to that of calicheamicinone) [for structure of esperamicin A₁, A₂, A_{1b} and X, see: Golik et al. J. Am. Chem. Soc.; 109:3461 (1987); Golik et al., J. Am. Chem. Soc., 109:3462; Konishi et al., J. Antibiotics, 1605 (1985)] by degradative means.

Thus, synthesis might be valuable in providing sharper insights into the functional sub-components of the enediyne drugs. Moreover the synthesis of either of the intact subunits as well as of the entire drug poses an obvious challenge to those who are sensitive to general issues of strategy and tactics in organic chemistry.

Not surprisingly then, a great deal of fascinating science has already issued from synthetic undertakings in this area [(a) Kende et al., Tetrahedron Lett., 29:4217 (1988); (b) Schreiber et al., Tetrahedron Lett., 30:433 (1989); Schoenen et al., Tetrahedron Lett., 30:3765 (1989); (c) Magnus et al., J. Chem. Soc., Chem. Commun., 916 (1989); Magnus et al., Tetrahedron Lett., 30:1905 (1989); Magnus et al., Tetrahedron Lett., 3637 (1989); (d) Tomioka et al., Tetrahedron Lett., 30:851 (1989); (e) Nicolaou et al., Angew. Chem. Int. Ed. Eng., 27:1097 (1988); and also see Nicolaou et al., J. Am. Chem. Soc., 110:4866 (1988) and Nicolaou et al., J. Am. Chem. Soc., 110:7247 (1988).

The present inventors and their co-workers have been involved in the enediyne problem at several levels. Early efforts led to the preparation of a functionalized core structure [Danishefsky et al., J. Am. Chem. Soc., 110:6890 (1988)] and to the synthesis of systems with suitable functionality to actuate both diyl formation and DNA cleavage [(a) Mantlo, et al., J. Org. Chem., 54:2781 (1989); Haseltine et al., J. Am. Chem. Soc., 111:7638 (1989)]. The first total synthesis of the aglycone of Compound **2** (i.e. calicheamicinone), derivatives and analogs thereof is disclosed hereinafter [we suggest this name which incorporates the standard suffix use to denote the aglycone substructure of the anthracycline antibiotics].

### Brief Summary of the Invention

The present invention is directed to calicheamicinone, the aglycone portion of calicheamicin, its derivatives and analogs, a pharmaceutical composition containing the same, a method for producing an intermediate useful in the preparation of one of the above compounds, as well as to particularly useful intermediates.

Calicheamicinone, analogs and derivatives thereof have a structure that corresponds to structural Formula I 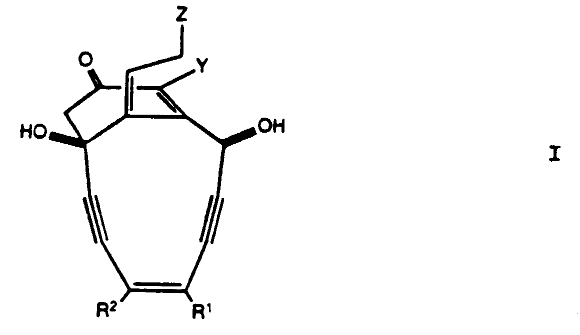
wherein R¹ and R² are the same or different and are selected from the group consisting of hydrogen, C₁-C₆ alkyl, phenyl, C₁-C₆ alkyl-substituted phenyl, benzyl and C₁-C₆ alkyl-substituted benzyl, or
R¹ and R² together with the intervening vinylene group form a phenyl group or a C₁-C₆ alkyl-substituted phenyl group;
Y is selected from the group consisting of hydrogen and NHR³ wherein R³ is selected from the group consisting of C₁-C₆ alkyl, C₁-C₆ alkanoyl, benzoyl, and carbonyl C₁-C₆ alkoxy; and
Z is selected from the group consisting of hydroxyl, C₁-C₆ acyloxy, C₁-C₆ acylthioxy, benzoyloxy, benzoylthioxy, and SSSR⁴ where R⁴ is C₁-C₆ alkyl or benzyl.

Calicheamicinone, where R¹ and R² are both hydrogen, Y is NHC(O)OCH₃ and Z is SSSCH₃, is most preferred. In particularly a preferred compound, R¹ and R² are both hydrogen, Z is hydroxyl, thioacetate, and more particularly SSSR⁴, where R⁴ is C₁-C₆ alkyl or benzyl, and Y is hydrogen or NHR³ where R³ is carbonyl C₁-C₆ alkoxy.

A pharmaceutical composition comprising a compound of Formula I as an active ingredient is also contemplated. The composition contains such a compound in an amount effective for a desired purpose such as cleaving DNA, as an antimicrobial agent, or as a cytotoxic (anti-tumor) agent. The compound is dissolved or dispersed in a physiologically acceptable diluent.

A particularly preferred intermediate has a structure corresponding to Formula V, below, where R¹ and R² are as described above, and R⁵ and R⁶ are the same or different and are hydrogen or C₁-C₃ alkyl, or R⁵ and R⁶ together with the intervening ethylene group comprise a 5- or 6-membered ring. R¹, R², R⁵ and R⁶ are each preferably hydrogen. 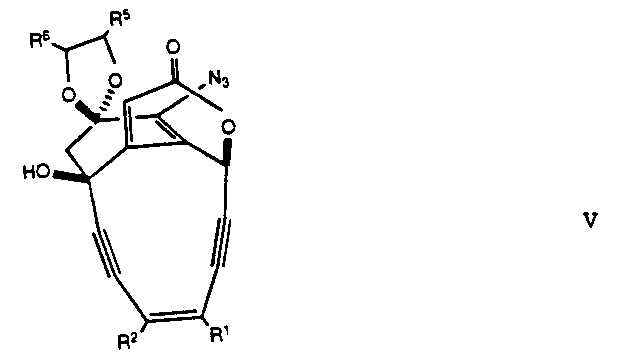
Another particularly preferred intermediate has a structure corresponding to Formula IX where R¹, R², R⁵, R⁶ and Y are as defined previously, and Z* is the before-defined group of Z substituent groups, further including mercaptan (SH). R¹, R², R⁵ and R⁶ are preferably hydrogen. 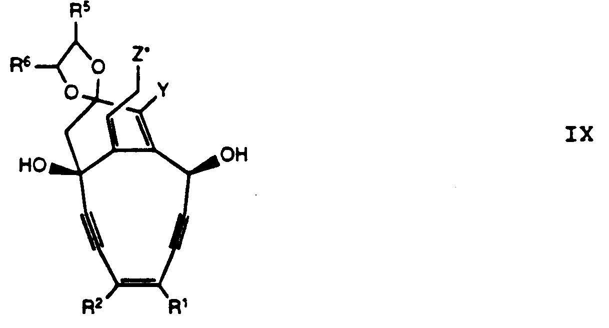
A method of forming a compound whose structure corresponds to that shown in Formula VI, below, is also contemplated, wherein R¹, R², R⁵ and R⁶ are as before defined, and are each preferably hydrogen. 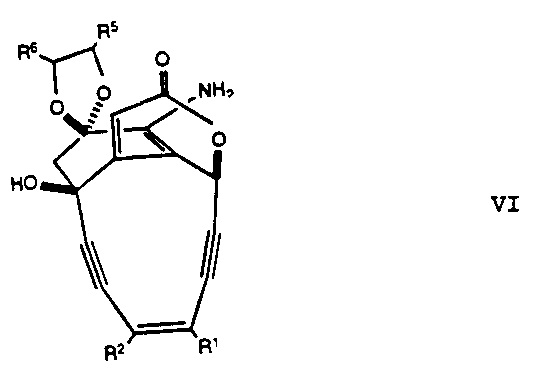
Here, a compound corresponding in structure to that shown in Formula VII is utilized, wherein R¹, R², R⁵ and R⁶ are as before, and X is halo. 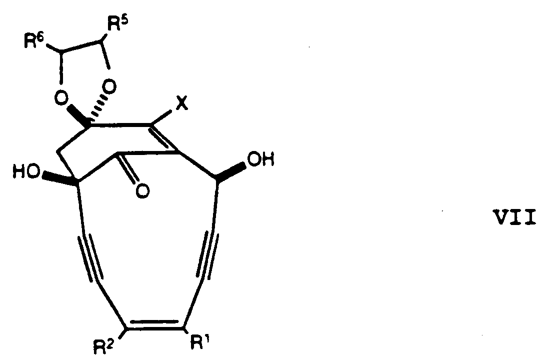
A compound of Formula VII is reacted:
(i) to replace the halo group with an azido substituent;
(ii) to esterify the allylic hydroxyl group to form an ester whose carboxyl portion has the formula -C(O)CH₂P(O)(OR⁷)₂, wherein R⁷ is C₁-C₆ alkyl as before defined; and
(iii) to carry out an intramolecular Emmons condensation of the formed ester to form a compound having structural Formula VIII, shown below

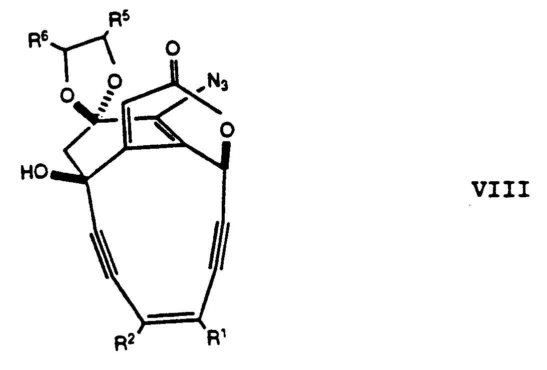

The resulting compound of Formula VIII is recovered, as preferably are intermediate product compounds formed in proceeding from a compound of Formula VII, and then reduced to form the corresponding compound of Formula VI. A compound whose structure corresponds to that shown in Formula VI can then be utilized to form a compound whose structure corresponds to that of Formula I.

### Brief Description of the Drawings

In the drawings forming a portion of this description,
Figure 1 illustrates the structural formulas for calicheamicin γ1 (Compound **1**) and for the aglycone portion thereof, calicheamicinone (Compound **2**). In Figure 1 and the other figures, Me is methyl and Et is ethyl.

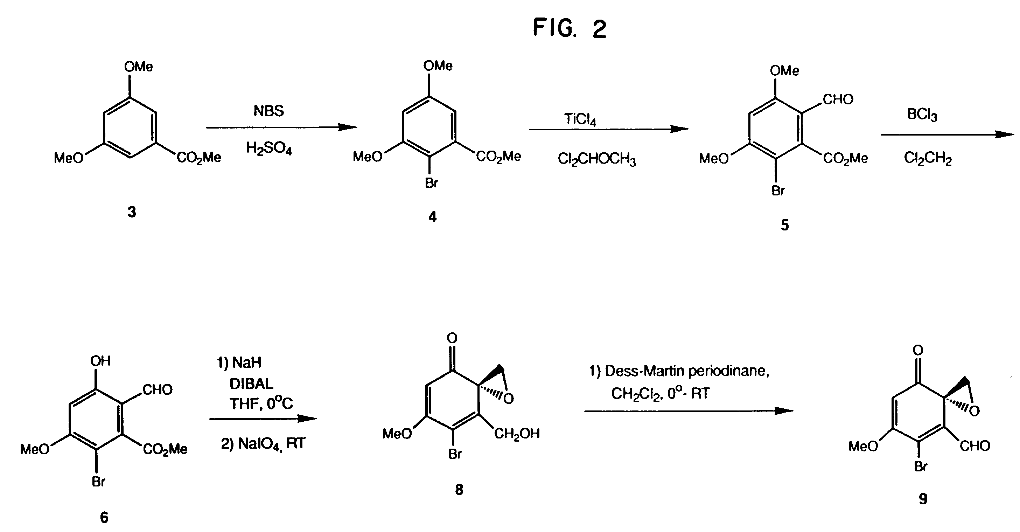

Figures 2 through 6 illustrate structural formulas of compounds and the reaction scheme described hereinafter to prepare calicheamicinone, beginning with commercially available Compound **3**. Chemical formulas for reagents, solvents and temperatures are shown above and below reaction arrows have their usual chemical meanings. Reagent abbreviations are used consistently throughout and are as follows: NBS is N-bromosuccinimide, DIBAL is disobutylaluminum hydride, THF is tetrahydrofuran, Dess-Martin periodinane is prepared as described in Dess et al., J. Org. Chem., 48:4155 (1983), 0°-RT means that a reaction was begun at zero degrees C and was completed at room temperature (RT), TMSTFA is trimethylsilyl trifluoroacetate, KHMDS is potassium hexamethyldisilazide, CSA is camphorsulfonic acid, KOAc is potassium acetate, HOAc is acetic acid, DMSO is dimethyl sulfoxide, Me is a methyl group (CH₃), MeOH is methanol, Pip is piperidine, Py is pyridine, ⁱPr is isopropyl, Ph is phenyl, and HSAc is thioacetic acid. Heavily blackened or blackened wedge-shaped lines indicate bonds that project above the plane of the page, where as dashes indicate bonds that project below the plane of the page. Hydrogen atoms bonded to ring carbons are not illustrated.

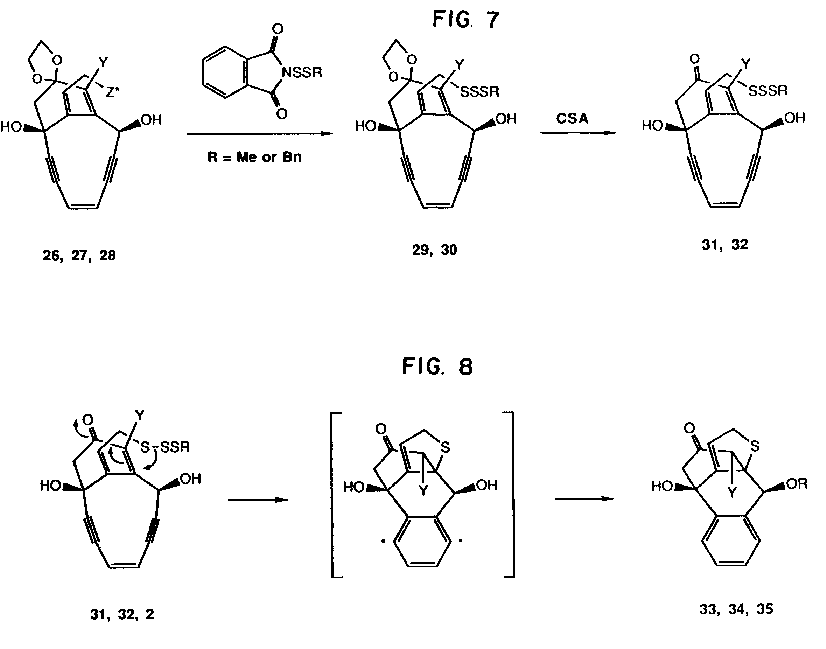

Figure 7 illustrates the synthesis of calicheamicinone analogs of the des-ureido series, starting with previously reported Compound **26**, which was first converted to Compound **27** (also previously reported), and then to Compound **28**. In each of Compounds **26** through **32**, Y is hydrogen. For Compound **26**, Z* is OH (hydroxyl); for Compound **27**, Z* is SAc (thioactyl); and for Compound **28**, Z* is SH (mercaptan). The "R" group of the phthalimidodisulfide is Me (methyl) for Compounds **29** and **31**, and Bn (benzyl) for Compounds **30** and **32**.
Figure 8 illustrates a Bergman type cyclization for Compounds **31**, **32** and **2**, with the proposed intermediate diradical shown in brackets followed by the observed Bergman product. Y and R for Compounds **31** and **32** are as discussed for Figure 7. Y is -NOCO₂CH₃ and R is Me for Compound 2. Y is hydrogen for Compounds **33** and **34**, whereas R is hydrogen in Compounds **33** and **35** and Ac (acetyl) in Compound 34. Y is NHCO₂CH₃ in Compound **35**. Arrows in the structures of Compounds **31**, **32** and **2** illustrate the proposed Michael addition of sulfur and the flow of electrons during the Bergman type cyclization.

The present invention has several benefits and advantages.

A salient benefit is that the extremely potent DNA cleaving calicheamicinone is prepared for the first time.

A particular advantage of the invention resides in the order in which the urethane group is introduced into the molecule relative to the trisulfide group.

Another benefit of the invention is compounds bearing an enediyne skeleton that also bear the trisulfide group and bridgehead Michael acceptor.

Still further benefits and advantages will be apparent to a skilled worker from the description that follows.

### Detailed Description of the Invention

The present invention relates to calicheamicinone, intermediates useful in preparing calicheamicinone, derivatives and analogs thereof, methods of making and using those materials, and compositions containing the same.

### I. Compounds, Compositions and Methods

Calicheamicinone, derivatives and analogs thereof are all considered compounds of the invention. A calicheamicinone derivative is defined as a compound having the structure of calicheamicinone plus one or more additional groups that replace a ring hydrogen, such as R¹ and R² in the structural formula below. A calicheamicinone analog has the basic structure of the calicheamicinone ring system, but the groups Y or Z (below) are different from those found in calicheamicinone, i.e., in which Y is NHC(O)OCH₃ and Z is SSSCH₃. A compound of the invention could be both a derivative and an analog by the above definitions, but will be discussed as being an analog for simplicity.

A compound of this invention has a structural Formula I shown below 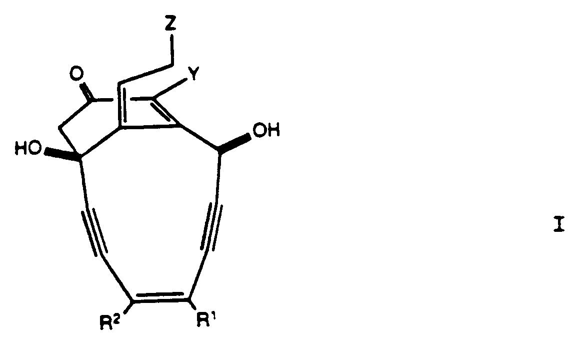
wherein R¹ and R² are the same of different and are selected from the group consisting of hydrogen C₁-C₆ alkyl, phenyl, C₁-C₆ alkyl-substituted phenyl, benzyl and C₁-C₆ alkyl-substituted benzyl,
or R¹ and R² together with the intervening vinylene group form a phenyl group or a C₁-C₆ alkyl-substituted phenyl group;
Y is selected from the group consisting of hydrogen and NHR³, wherein R³ is selected from the group consisting of C₁-C₆ alkyl, C₁-C₆ alkanoyl, carbonyl C₁-C₆ alkoxy and benzoyl; and
Z is selected from the group consisting of hydroxyl, C₁-C₆ acyloxy, C₁-C₆ acylthioxy, benzoyloxy, benzoylthioxy, and SSSR⁴ where R⁴ is C₁-C₆ alkyl or benzyl.

In the structure above and all structures illustrated herein, heavily darkened or darkened, wedge-shaped lines illustrate bonds that project above the plane of the page, whereas dashes illustrate bonds that project below the plane of the page. In addition, hydrogen atoms bonded to ring carbon atoms are not shown so that the other structures can be more easily seen.

In the above formula, and the other structural formulas and their underlying compounds R¹ and R², C₁-C₆ alkyl groups are illustrated by methyl, ethyl, iso-propyl, butyl, iso-butyl, pentyl, hexyl, 2-methylpentyl and the like. Those same C₁-C₆ alkyl groups can also be substituted in C₁-C₆ alkyl-substituted phenyl and benzyl groups.

The same C₁-C₆ alkyl groups can be present as R³ in the Y substituent. When R³ is a C₁-C₆ alkanoyl or benzoyl group, Y is an amide and the carboxylic acid portion of that amide (the C₁-C₆ alkanoyl group) is the carbonyl-containing residuum of a carboxylic acid corresponding to an appropriate before-described C₁-C₆ alkyl group. Illustrative C₁-C₆ alkanoyl groups include formyl, acetyl, propionyl, iso-butyryl, pentanoyl, hexanoyl and the like.

When R³ is a carbonyl C₁-C₆ alkoxy group, Y is a urethane. Here, a C₁-C₆ group can be the carbon-containing portion of a before-described C₁-C₆ alkyl group that is bonded through an oxygen atom to the urethane carbonyl group. Exemplary C₁-C₆ alkoxy groups include methoxy, ethoxy, iso-propoxy, iso-butoxy, pentyloxy, hexyloxy, 2-methylpentyloxy, and the like.

The Z group in the above structural formula is an alcohol, an ester, their respective sulfur-containing analogs or an alkyl or benzyl trisulfido group. Thus, a C₁-C₆ acyloxy group is an ester that contains a before-described C₁-C₆ alkanoyl group with an oxygen atom linked to the ring structure. Exemplary groups include formyloxy, acetoxy, propionoxy, iso-butyroyloxy, pentanoyloxy, hexanoyloxy, and the like. Exemplary sulfur-containing analog esters (C₁-C₆ acylthioxy or benzoylthioxy or -S-C₁-C₆ alkanoyl or -S-benzoyl groups) include thioformyloxy, thioacetoxy, thiopropionoxy, thio-iso-butyroyloxy, thiopentoyloxy, thiohexanoyloxy, and the like. A C₁-C₆ alkyl R⁴ group is as discussed before.

In preferred practice, both R¹ and R² are hydrogen. In one group of more preferred compounds, Y is hydrogen, whereas in a still more preferred group of compounds, Y is NHR³ and R³ is carbonyl C₁-C₆ alkoxy.

Structural formulas for preferred compounds where R¹ and R² are both hydrogen (Formula II), as well as where Y is hydrogen (Formula III) and where Y is NHR³ (Formula IV) are shown below. 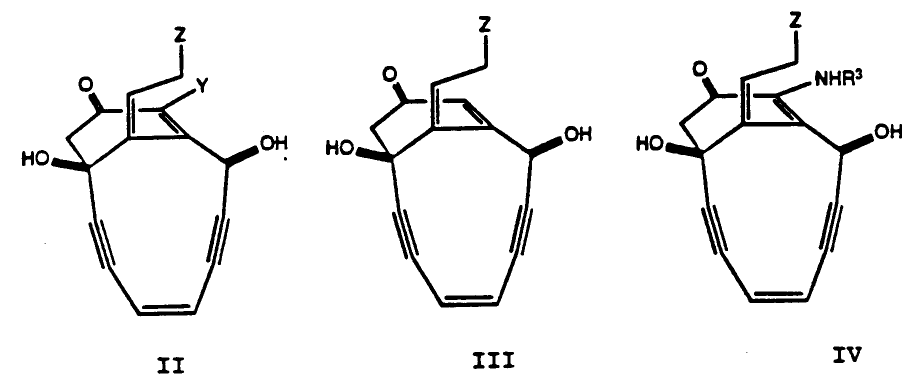
Particularly preferred compounds contain a Z group that is hydroxyl (OH), thioacetate [SC(O)CH₃] and more particularly SSSR⁴, where R⁴ is C₁-C₆ alkyl, particularly methyl, or benzyl. In these compounds, Y is preferably hydrogen or an NRH³ urethane group as discussed previously.

Most preferred is calicheamicinone (Compound **2**), whose structural formula is illustrated in Figure 1.

A compound of the invention is useful as a DNA cleaving agent as well as an antimicrobial and a cytoxic (antitumor) agent, as is calicheamicin (Compound **1**) itself. DNA cleavage can be assayed using the techniques described by Mantlo et al., J. Org. Chem.; 54:2781 (1989); Nicolaou et al., J. Am. Chem. Soc., 110:7247 (1988) or Zein et al., Science, 240:1198 (1988) and the citations therein. Anti-microbial and anti-tumor assays can be carried out by techniques described in U.S. Patent No. 4,837,206.

The before-described compounds can also be shown to undergo a Bergman cycloaromatization reaction in the presence of benzyl mercaptan, triethylamine and 1,4-cycloxadiene as discussed in Haseltine et al., J. Am. Chem. Soc., 111:7638 (1989). This reaction forms a tetracyclic reaction as is formed during DNA cleavage, and can be used as a co-screen to select more active compounds.

A pharmaceutical composition is also contemplated that contains calicheamicinone, a derivative or analog as active agent. Here, the compound is dissolved or dispersed in a physiologically acceptable diluent such as water, water/ethanol, normal saline, or a buffered aqueous solution, such as phosphate-buffered saline, or within vesicles as are well known. Exemplary further liquid diluents can be found in Remmington's Pharmaceutical Sciences, Mack Publishing Co., Easton, PA (1980). Solid dispersions including well known enteric capsules that pass through the stomach into the intestinal tract where they release a before-described compound to be sorbed into the body of a mammalian host are also contemplated.

A compound of the invention is present in such a pharmaceutical composition in an amount effective to achieve the desired result. For example, where in vitro DNA cleavage is the desired result, a compound of the invention can be utilized in an amount sufficient to provide a concentration of about 1.0 to about 500 micromolar (µM) with a DNA concentration of about 0.02 µg/µL. As a cytoxic (anti-tumor) agent, an effective amount of a compound of the invention is about 0.1 to about 15 µg per kilogram of body weight. A compound of the invention exhibits antimicrobial activity in a concentration range of about 0.01 to about 50 µg/mL. The above concentrations and dosages vary with the particular compound of the invention utilized as well as with the target, e.g., DNA, tumor, microbe, as is well known.

Calicheamicinone or a before-discussed derivative or analog thereof can also be utilized when linked to one or more sugar rings. As can be seen from Figure 1, calicheamicin itself contains an extremely complex side chain that contains several linked, derivatized sugar rings and an aromatic ring.

As noted before, calicheamicinone, a derivative or analog thereof is active itself as a DNA cleaving reagent, a cytotoxic agent and does not require a derivatized sugar moiety for its activity. However, the presence of one or more sugar rings linked to a hydroxyl group of an above compound can assist in dissolution or dispersion in aqueous media, including plasma. In addition, the presence of one or more linked sugar rings can help direct the active DNA cleaving agent to intracellular DNA or provide an oligonucleotide recognition device [Hawley et al., Proc. Natl. Acad. Sci. U.S.A., 86:1105 (1989).

Also contemplated herein is a chimeric drug that is the reaction product of calicheamicinone, a derivative or analog thereof and a carbohydrate side chain other than the carbohydrate chain present in calicheamicin. Exemplary carbohydrate side chains are daunosamine that can be obtained from daunorubicin and doxorubicin, glucosamine, ribose and arabinose. Oligosaccharide carbohydrate side chains include esperimicin trisaccharide, as well as the oligosaccharides prepared as disclosed in Halcomb et al., J. Am. Chem. Soc., 111:6661 (1989) and Friesen et al., J. Am. Chem. Soc., 111:6656 (1989), in which the glycoside-forming sugar ring is in a 1-hydroxyl form rather than an epoxide or glycal form, and from which the hydroxyl blocking groups have been removed. Those carbohydrate side chains can be cleaved from the aglycone portions (where necessary), and linked to a compound of the invention by the method discussed in Schmidt, Angew Chem. Int. Ed. Eng., 25:212 (1986).

The reaction scheme utilized to obtain calicheamicinone is illustrated in Figures 2-6 and is discussed in greater detail hereinafter in the section entitled "Results".

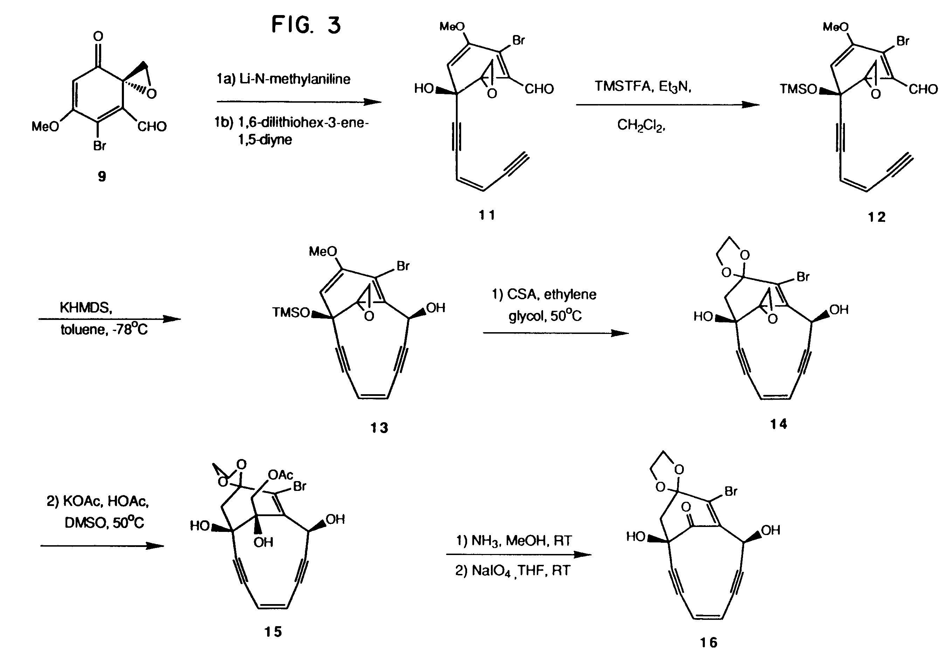

That reaction scheme is also useful in preparing the calicheamicinone derivatives discussed above. For example, a compound wherein R¹ and R² are other than hydrogen can be prepared in a manner analogous to the steps for the preparation of Compounds **3 - 9** as shown in Figure 2. Thereafter, an enediyne substituted at one or both positions of the vinylene group is utilized in place of the unsubstituted enediyne shown in Figure 3 for the conversion of Compound **9** to Compound **11**. The remainder of that reaction sequence is thereafter followed.

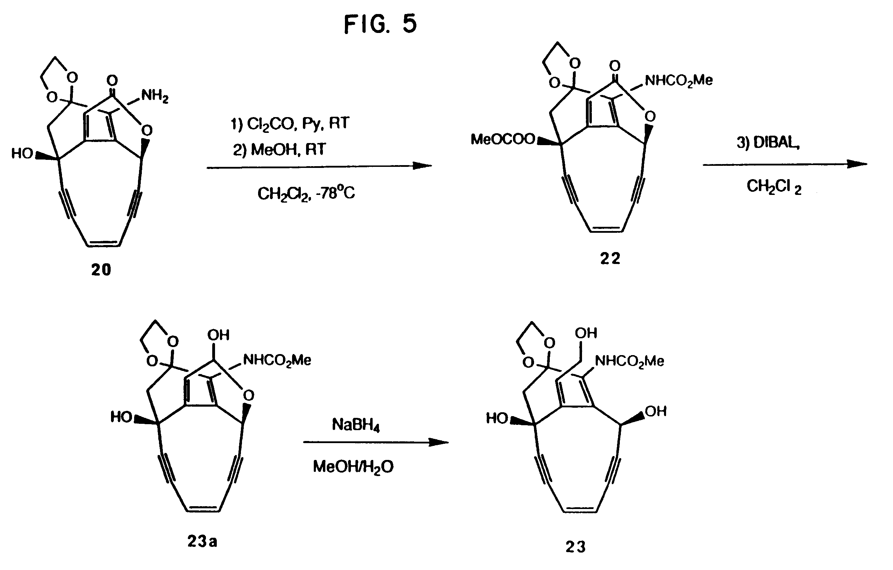

Where a C₁-C₆ alkyl group is desired for R³ of the Y substituent, the amine group of Compound **20** shown in Figure 5 or a derivative of that compound can be reductively alkylated using the desired alkyl aldehyde and a borohydride reagent such as sodium cyanoborohydride. Where R³ is a carbonyl C₁-C₆ alkoxy group other than methanol, a desired alcohol other than methanol is utilized in the second step of Figure 5 between Compounds **20** and **22**. Use of a suitable anhydride or acid halide reagent can convert Compound **20** into an ester-amide analogous to Compound **22** that can be selectively cleaved to yield the desired amide. Compounds in which Y is hydrogen and Z is hydroxyl and thioacetyl are disclosed in Haseltine et al., J. Am. Chem. Soc., 111:7638 (1989) and also hereinafter.

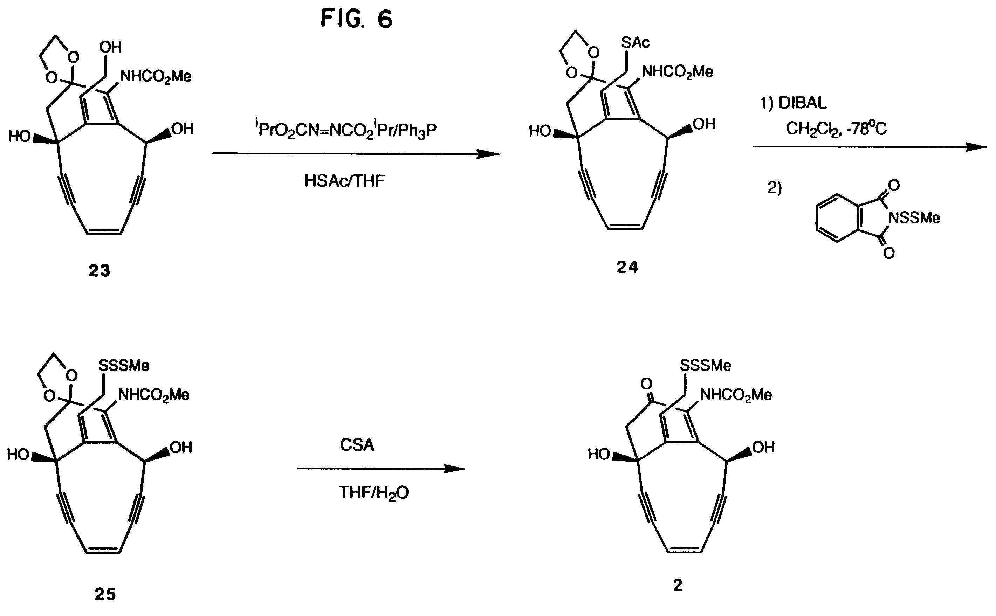

Synthesis of several exemplary Z groups is illustrated hereinafter, and should otherwise be apparent to a skilled worker. For example, Compound **23** in which Z is hydroxyl can be deblocked using camphorsulfonic acid as illustrated herein, as can Compound **24** to form a calicheamicinone derivative or analog. C₁-C₆ Acyloxy and C₁-C₆ acylthioxy compounds can be prepared from Compound **23** by direct acylation, or reaction with a different thioacid as shown in the first reaction of Figure 6, respectively. Choice of an R⁴ group where Z is SSSR⁴ is made by selecting the mercaptan utilized in forming the phthalimido reagent used to convert Compound **24** into a compound analogous to Compound **25**.

As noted elsewhere herein, the time or position of forming the urethane group, when present, relative to forming a trisulfide or other Z group is of import. The bridgehead enone present in a compound such as Compound **16** or derivative can provide the necessary enolate stabilization to support an addition-elimination reaction used to add the azido group. Thereafter, formation of a conjugated lactone such as Compound **19** provides a sufficiently stable environment for transformation of the azide into a urethane, followed by a series of reactions that lead to introduction of the Z group, and the final deblocking step in which calicheamicinone or a before-described derivative or analog is formed.

Compound **19** and its derivatives are thus key intermediates in the formation of calicheamicinone or its derivatives and analogs. A generic structural formula that includes Compound **19** and its appropriate derivatives is shown below in Formula V. 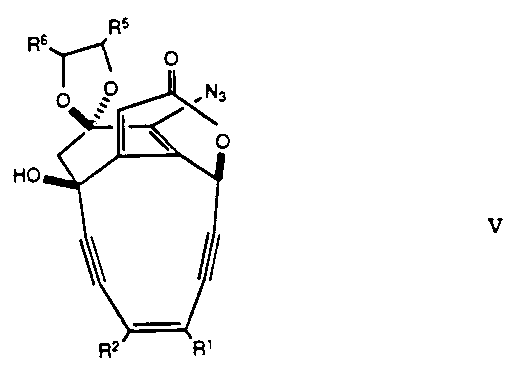
In the above formula, R¹ and R² are as described previously, and R⁵ and R⁶ are the same or different and are hydrogen or C₁-C₃ alkyl, or R⁵ and R⁶ together with the depicted ethylene group comprise a 5-or 6-membered ring. Thus, R⁵ and R⁶ can each be methyl, ethyl, propyl or iso-propyl or R⁵ and R⁶ together with the intervening two carbons of the ethylene group form a cyclopentane or cyclohexane ring.

As is apparent from the structural formula, above, the dioxygen-containing spiro ring system that includes R⁵ and R⁶ is a ketal structure formed from the keto group of the large ring system and a diol. Exemplary diols include ethylene glycal (where R⁵ and R⁶ are hydrogen), propylene glycol, 1,2-butanediol, 2,3-butanediol, 1,2-pentanediol, and 3,4-hexanediol, as well as 1,2-cyclopentanediol and 1,2-cyclohexanediol when R⁵ and R⁶ and the intervening ethylene form a 5- or 6-membered ring, respectively.

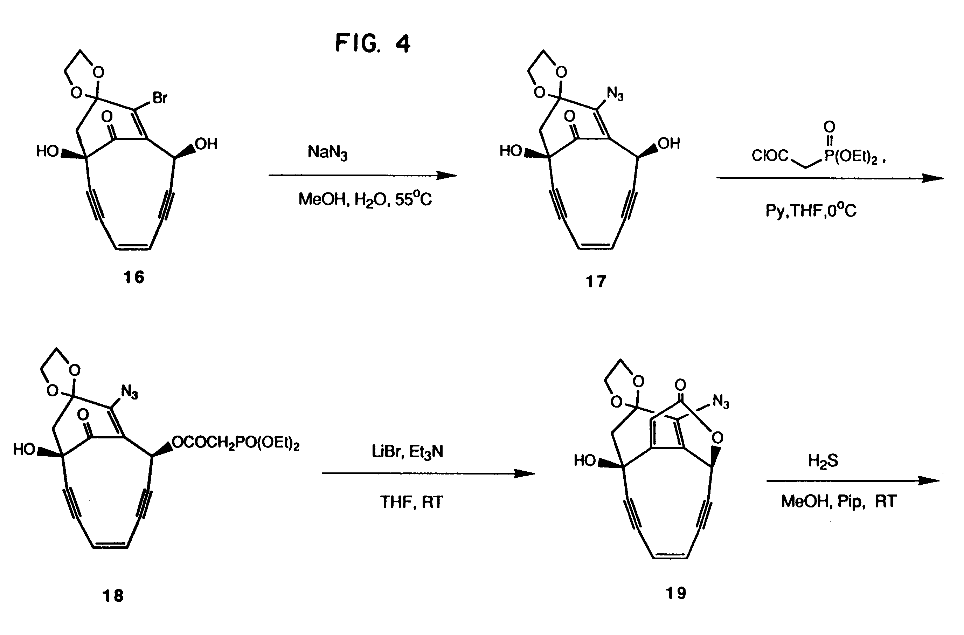

In preferred practice, R¹ and R² are both hydrogen and R⁵ and R⁶ are the same. Most preferably, both R⁵ and R⁶ are hydrogen, and the compound of the formula above is Compound **19** shown in Figure 4.

An above compound such as Compound **19** plays an important role in the preparation of a urethane-containing calicheamicinone derivative or analog. A precursor to such a urethane derivative is an amino compound whose generic chemical formula is shown below in Formula VI, wherein R¹, R², R⁵ and R⁶ are as defined before. 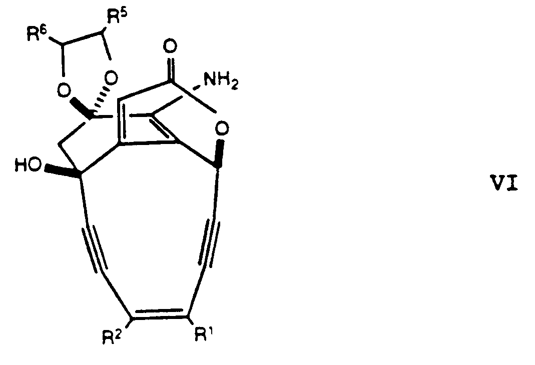
A method of preparing a compound of the above structural formula constitutes another aspect of this invention. Here, a compound having the structure of Formula VII, below, wherein R¹, R², R⁵ and R⁶ are as before defined and X is a halo group (fluoro, chloro, bromo or iodo) is reacted 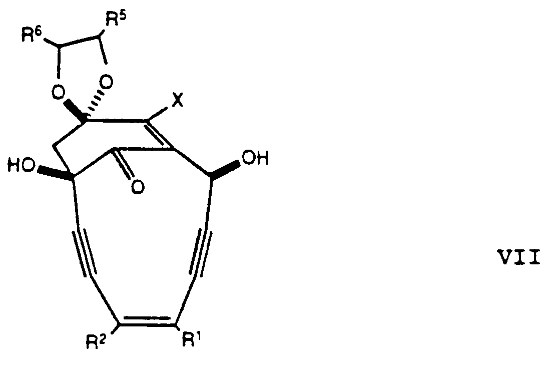
(i) to replace the halo group with an azido substituent;
(ii) to esterify the allylic hydroxyl group to form an ester whose carboxyl portion has the formula -C(O)CH₂P(O)(OR⁷)₂, wherein R⁷ is C₁-C₆ alkyl as before defined; and
(iii) to carry out an intramolecular Emmons condensation of the formed ester to form a compound having structural Formula VIII, shown below 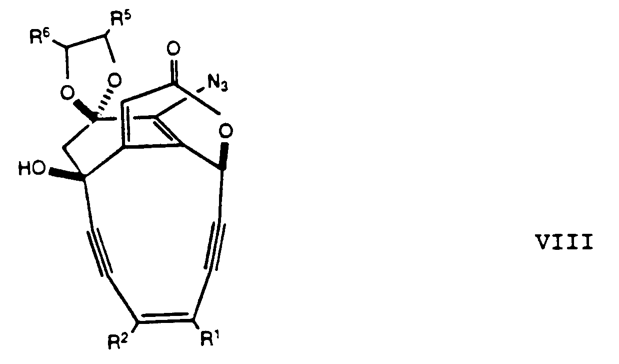
The compound so prepared is recovered, and then reduced to form the corresponding amino compound. The formed amino compound is preferably recovered, as are the products of each sub-step. That amino compound can then be utilized to form an intermediate from which a calicheamicinone derivative whose Y group is NHR³, as before defined.

The previously noted preferences as to R¹, R², R⁵ and R⁶ hold for the compounds utilized in this method. The halo group, X, is preferably bromo, and that halo group is typically replaced by an azido group using an alkali metal azide such as sodium or potassium azide. R⁷ is preferably methyl or ethyl. A corresponding acid halide is preferably the source of the -C(O)CH₂P(O)(OR⁷)₂ group.

The reduction of the azido group to the corresponding amine can be carried out with substantially any reducing agent that will not attack the other double or triple bonds in the molecule. Hydrogen sulfide is an exemplary reducing agent.

Sub-steps (ii) and (iii), above, must be carried out with sub-step (iii) following sub-step (ii). However, sub-step (i) can follow sub-step (ii) or sub-step (iii). In preferred practice, sub-steps (i), (ii) and (iii) are carried out in the order enumerated above.

Another important intermediate herein is a compound having the generic structural formula shown in Formula IX, below, where R¹, R², R⁵, R⁶ and Y are as before defined, and Z* is the previously defined Z group that further includes mercaptan (SH). 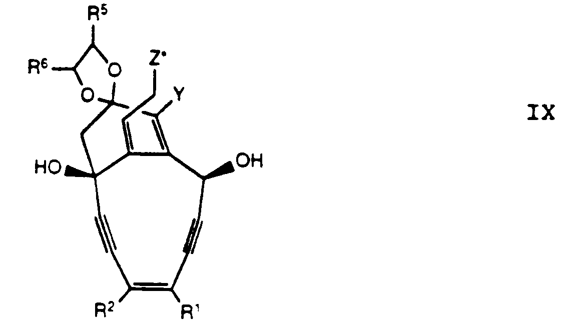
Specific, particularly preferred, compounds within the above generic formula include Compounds **23**, **24**, **25**, **26**, **27**, **28**, **29** and **30** wherein R¹, R², R⁵ and R⁶ are hydrogen. Removal of the ketal protecting group can provide a compound of this invention.

A compound of the above generic formula can be prepared as described herein. Other routes of preparation are also available. For example, a bridgehead allylic alcohol function; i.e., where Z* is hydroxyl, can be introduced into the basic ring structure of a compound such as Compound **16** or Compound **17**, or an analog thereof, using the method described in Magnus et al., Chem. Commun., 1989:916 for a similar enediyne. The resulting bridgehead allylic alcohol can thereafter be utilized as is or reacted further as discussed herein or in the above Magnus et al. article.

Preferred members of the group of compounds encompassed by structural Formula IX are the allylic alcohols whose structures are illustrated in structural Formulas X and XI, below, wherein R¹, R², R⁵, R⁶ and Y are as defined before. Compounds having the structures of Formulas X and XI include some of those particularly preferred compounds discussed above. 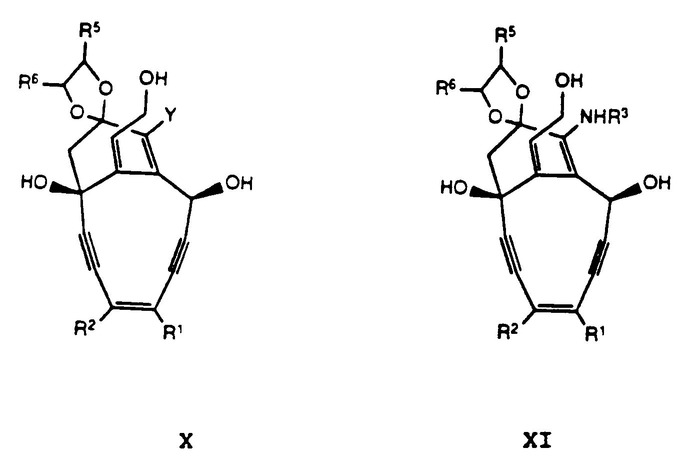
Calicheamicinone, an analog, a derivative and most of the intermediates in the preparation thereof are prepared as racemic mixtures (modifications). Such racemates are preferably separated into their component enantiomers (d and l or R and S forms) prior to use in a pharmaceutical preparation.

Resolution, where desired, can take place at substantially any time during the synthesis. However, resolution can conveniently be carried out using a compound whose structure corresponds to that of Formula X.

In an exemplary resolution, a compound whose structure corresponds to Formula X is esterified with one or the other enantiomers of a carboxylic acid such as (R)-(-)-mandelic acid or (S)-(+)-mandelic acid, and the resulting ester is resolved by usual means. Alternatively, an alcohol corresponding in structure to Formula X can be esterified as with phthalic anhydride to form the corresponding half-ester. That half-ester can then be resolved by usual means via salt formation with a chiral amine such as brucine.

### II. Results - Preparation of Calicheamicinone

The general synthetic plan for the synthesis of calicheamicinone was drawn from earlier work on simpler systems. However it was necessary to provide the means to introduce the urethane function at the bridgehead double bond. The optimal timing for this installation emerged as a serious problem. The solution is described below, and illustrated in Figures 2-6.

Commercially available ester Compound **3** underwent regiospecific bromination (NBS, H₂SO₄; DMF) to afford Compound **4** which upon formulation (Cl₂CHOCH₃; TiCl₄) gave Compound **5**. The aldehyde function was employed to direct regiospecific mono-demethylation (via BCl₃), giving rise to the required phenol Compound **6** (65 percent from Compound **3**). The sodium salt of Compound **6** was subjected to reduction to provide the unstable triol Compound **7**, which, upon treatment with sodium periodate afforded Compound **8** [Becker et al., Tetrahedron Lett. 4205 (1972)]. Upon oxidation of crude Compound **8** with the Dess-Martin periodinane [Dess et al., J. Org. Chem., 48:4155 (1983)], there was obtained the spiroepoxyaldehyde Compound **9**.

The yield for the three steps from Compound **6** to Compound **9** in large scale was about 40 percent. This reaction sequence from Compound **3** through Compound **9** is illustrated in Figure 2.

The next phase of the effort involved insertion of the six carbon enediyne bridge between the ketone and aldehyde functions. Dilithioenediyne Compound **10** [Danishefsky et al., Tetrahedron Lett., 29:4681 (1988)] was added to the ketone in the nominal presence of the aldehyde using the logic of in situ protection as developed, in another context, in the pioneering research of Comins [Comins et al., Tetrahedron Lett., 23:3979 (1982); Comins et al., J. Org. Chem., 44:1078 (1984)]. Reaction of Compound **9** with Compound **10** in the presence of lithium N-methylanilide afforded Compound **11**.

Silylation of the tertiary alcohol gave rise to Compound **12** which on cyclization (potassium-3-ethyl-3-pentoxide) [Brown et al., J. Am. Chem. Soc., 75:4112 (1953)] provided the core system Compound **13** (about 35-40 percent overall yield for the three steps from Compound **9** on a 2-gram scale). No stereoisomer of the secondary alcohol was observed.

After considerable experimentation it was found that the enol ether function was not suitable for the required subsequent manipulations. Accordingly, Compound **13** was converted to ketal Compound **14** (CSA-ethylene glycol 89 percent yield). Acetolysis of the epoxide (KOAc; AcOH; DMSO) led to crude Compound **15** which, upon de-acylation (NH₃; MeOH) and oxidation (sodium periodate) gave rise to ketone-ketal Compound **16** (83 percent combined yield).

The reactions from Compound **9** through Compound **16** are illustrated in Figure 3.

The bridgehead enone of Compound **16** presented a target of opportunity for the introduction of an azido function. For this to be possible the ketone at the one carbon bridge had to provide adequate enolate stabilization to support an addition elimination mechanism - a possibility presaged by the research of Magnus [Magnus et al., J. Chem. Soc., Chem. Commun., 916 (1989); Magnus et al., Tetrahedron Lett., 30:1905 (1989); Magnus et al., Tetrahedron Lett., 3637 (1989)].

Reaction of Compound **16** with sodium azide in methanol afforded an 82 percent yield of Compound **17**. As matters transpired this stage was still too early to actually unveil the urethane. First, the secondary alcohol was acylated (EtO)₂P(O)CH₂COCl; pyridine) [the diethylphosphonyl acetyl chloride was obtained from the corresponding acid; Cook et al., Synthesis, 283 1981)], and the resultant ester Compound **18** was subjected to intramolecular Emmons condensation [Haseltine et al, J. Am. Chem. Soc., 111:7638 (1989); Rathke et al., J. Org. Chem., 50:2629 (1985)] to produce Compound **19** (50 percent from Compound **17**).

The reaction sequence from Compound **16** through Compound **19** is shown in Figure 4.

The conjugation afforded by the conjugated lactone provided a sufficiently stable setting for the steps required to transform the azide to the methyl carbamate function. Reduction of Compound **19** (H₂S-piperidine-methanol; 95 percent yield) led to the remarkably robust vinyl amine Compound **20**. The latter, upon treatment with phosgene in pyridine, gave rise to a presumed bis acylation product, Compound **21** and thence with methanol and pyridine to the carbamate-carbonate Compound **22** in 80 percent overall yield. Compound **20** was also converted to Compound **22** by reaction with trichloromethyl chloroformate to presumably form the bis acylation product which was then converted to Compound **22** by reaction with pyridine and methanol. In this reaction sequence, the yield was 78 percent from Compound **20**.

Treatment of Compound **22** first with DIBAL (which results in deprotection of the tertiary alcohol and reduction of the lactone to a lactol) to form Compound **23a**, followed by sodium borohydride, produced the alcohol Compound **23** in 43 percent overall yield. A 51 percent yield of Compound **23** from Compound **22** was subsequently obtained. The synthetic steps from Compound **19** through Compound **23** are shown in Figure 5.

The first sulfur atom was installed by a Mitsunobu reaction on Compound **23** (thiolacetic acid, triphenylphosphine, di-isopropylazodicarboxylate) to produce Compound **24** (about 45 percent yield) [Volante, Tetrahedron Lett., 22:3119 (1981)]. This reaction is badly complicated by the formation of a roughly equal amount of the cyclic (six membered ring) ether.

Treatment of thioacetate Compound **24** with DIBAL resulted in de-acetylation. The crude product (presumably the allylic aluminum thiolate) was subjected to the action of methyl phthalamido disulfide, [the first synthesis of an allylic trisulfide in this general series was accomplished by Magnus and co-workers; Magnus et al., J. Chem. Soc., Chem. Commun., 916 (1989); Magnus et al., Tetrahedron Lett., 30:1905 (1989); Magnus et al., Tetrehedron Lett., 3637 (1989)] thereby leading to trisulfide Compound **25** (65 percent from Compound **24** when carried out in a small scale synthesis, with about 46 percent when done on a 12 mg scale).

Finally, the ketal linkage was cleaved through the action of CSA in aqueous THF at room temperature. There was thus obtained dl-calicheamicinone (Compound **2**) as a powder in 65 percent yield. While there exists, to our knowledge, no reference sample of this compound, (Compound **2**) the structure proposed here is firmly supported by infrared NMR and mass spectral determinations. Furthermore the assignments are supported by the close similarity of these compounds with those of the des-ureido series which were in turn supported by crystallographic determinations [Danishefsky et al., J. Am. Chem. Soc., 110:6890 (1988); Mantlo et al., J. Org. Chem. 54:1781 (1989); Haseltine et al., J. Am. Chem. Soc., 111:7638 (1989)]. The synthetic steps from Compound **23** to Compound **2** are illustrated in Figure 6.

Calicheamicinone analogs have been prepared where Y in the prior generic formula is hydrogen; i.e., the des-ureido calicheamicinones, that undergo a Bergman type cyclization reaction in the presence of a reducing agent and 1,4-cyclohexadiene. Syntheses for these compounds have been published in part in Haseltine et al., J. Am. Chem. Soc., 111:7638 (1989).

These compounds can also be prepared following the generalized reaction scheme shown in Figures 2-6, without the steps required for introduction of the ureido group. A simplified reaction scheme for the preparation of these materials from the allylic alcohol Compound **26** is illustrated in Figure 7. The syntheses of Compound **26** is itself described in Haseltine et al., J. Am. Chem. Soc., 111:7638 (1989), whose disclosures are incorporated by reference.

Thus, thioacetate Compound **27**, previously obtained from the corresponding primary mesylate, [Haseltine et al., J. Am. Chem. Soc., 111:7638 (1989)] has subsequently been prepared directly in about 60 percent yield from triol Compound **26** using the conditions of Volante as described for the preparation of Compound **24**. Deacylation (DIBAL, CH₂Cl₂,-78°C) followed by treatment with methyl phthalimido disulfide (CH₂Cl₂, THF, RT) [Magnus et al., Chem. Commun., 916 (1989)] or benzyl phthalimido disulfide [Harpp et al., Int. J. Sulfur Chem., 1:57 (1971)] gave the corresponding alkyl trisulfides, Compound **29** and Compound **30**, in 84 and 61 percent yields, respectively. Deprotection of the bridgehead enone by exposure of Composed **29** and Compound **30** to the action of camphorsulfonic acid (CSA) in THF/H₂O at room temperature provided the derivative enones Compound **31** and Compound **22** in quantitative yield.

Interestingly, the syntheses of trisulfide Compounds **29**, **30** and **25** (discussed before) are apparently not complicated by formation of any corresponding disulfide. The factors responsible for the difference between our findings and those reported by Magnus et al., Chem. Commun., 1989:916 where di- and tetrasulfides were reported have not been clarified. It must be that subtle differences in the nature of the substrate undergoing thiylation determine the course of cleavage of the Harpp disulfides. Alternatively the difference might arise from the method in which the reagents are prepared and used [a detailed procedure for the preparation of methyl phthalimido disulfide is provided hereinafter].

With the allylic trisulfides (Compounds **31**, **32** and **2**) in hand, it was of interest to study their ability to potentiate the diyl-forming cascade. Treatment of either Compounds **31** or **32** with benzyl mercaptan, triethylamine, and 1,4-cyclohexadiene in methanol resulted in essentially complete conversion after two hours at room temperature, resulting in the formation in each instance of an approximately 50 percent yield of cycloaromatizied tetracycle Compound **33**.

In addition to routine characterization, the structure of Compound **33** was corroborated through acetylation of the secondary alcohol (Ac₂O, pyridine, RT) to provide the previously identified ester Compound **34** [Haseltine et al., J. Am. Chem. Soc., 111:7638 (1989). Similarly, the cycloaromatization of Compound **2** with the intact urethane afforded a 16 percent yield of Compound **35**, wherein the stereochemistry at C₁₀, the position of the ureido group, has not been rigorously determined. ¹H NMR data in hand are consistent with a structural assignment for Compound **35** in which the urethane function is of the α configuration. The same stereochemistry has been suggested for the Bergman product derived from calicheamicin γ₁. See: Ellestad et al., Tetrahedron Lett., 30:3033 (1989). These Bergman type cyclization reactions are illustrated in Figure 8.

In summary, these results demonstrate the clean introduction of the trisulfide trigger in extensively functionalized versions of the calicheamicin/esperamicin antibiotics. Moreover, thiol-induced cleavage in these systems (a model for the possible action of glutathione) initiates the cascade which culminates in Bergman cyclization. The urethane functionality of the natural products was not found to be a crucial ingredient of the substrate formula leading to a Bergman type cyclization product.

### Best Mode for Carrying Out the Invention

### Example 1: Compound 4

An acetonitrile solution [700 milliliters (mL)] of Compound **3** [102 grams (g), 0.52 moles (mol)] was treated with N-bromosuccinimide (NBS) (111 g, 0.62 mol) in one portion at zero degrees C and the reaction mixture was stirred overnight (about 15-18 hours) at room temperature. Saturated Na₂SO₃ solution (300 mL) was added, and most of the acetonitrile was removed under vacuum. The products were extracted with ether (4 x 500 mL) and the combined extracts were dried over MgSO₄. After most of the solvent was removed, the remainder was filtered and concentrated under vacuum. The residue was distilled (144-149 degrees C/1mmHg) to give 101 g (71 percent) of Compound **4** as a colorless oil, which slowly solidified on standing; m.p. 57-59 degrees C.

¹H NMR (250 MHz, CDCl₃) δ 6.81 (d, 1H, J = 2.7 Hz, CH arom.); 6.59 (d. 1H, J = 2.7 Hz, CH arom.); 3.94 (s, 3H, OCH₃); 3.90 (s, 3H, OCH₃); 3.83 (s, 3H, OCH₃). IR (CDCl₃) 2954, 1732, 1587, 1453, 1341, 1212, 1164, 1057 cm⁻¹. EIHRMS calcd for C₁₀H₁₁BrO₄ (M+2) 275.9820, found 275.9814. Anal. calcd. for C₁₀H₁₃BrO₄: C, 43.64; H, 4.03; Br, 29.06. Found: C, 43.38; H, 4.00; Br, 29.35.

### Example 2 : Compound 5

To a CH₂Cl₂ solution (500 mL) of Compound **4** (54.8 g, 0.20 mol) was added a solution of TiCl₄ (44 mL, 0.40 mol) in CH₂Cl₂ (200 mL) at -25 degrees C over 20 minutes. After the mixture was stirred at this temperature for 10 minutes, Cl₂CHOCH₃ (25 mL, 0.28 mol) was slowly added over 25 minutes at -20 degrees C, and the mixture was stirred at this temperature for 30 minutes. Aqueous HCl (1N, 150 mL) was slowly added to the mixture at -20 degrees C, followed by the addition of H₂O (500 mL). The aqueous layer was extracted with CH₂Cl₂ (3 x 500 mL) and the combined organic layers were dried over MgSO₄. After evaporation of the solvent, the crude solid was crystallized from ethyl acetate-tethrahydrofuran (EtOAc-THF) to give 47.8 g of Compound **5**. Concentration of the mother liquor and chromatography gave an additional 3.8 g of Compound **5** as a white solid (51.6 g, 85 percent overall); m.p. 187-188 degrees C.

¹H NMR (250 MHz, CDCl₃) δ 10.23 (s, 1H, CHO); 6.51 (s, 1H, CH arom.); 4.01 (s, 3H, OCH₃); 4.00 (s, 3H, OCH₃); 3.98 (s, 3H, OCH₃). IR (CDCl₃) 2954, 1740, 1682, 1598, 1343, 1215, 1055 cm⁻¹. EIMS m/z 302 (M⁺). EIHRMS calcd for C₁₁H₁₁BrO₅ (M⁺) 301.9789, found 301.9786.

### Example 3: Compound 6

To a CH₂Cl₂ suspension (260 mL) of Compound **5** (52.3 g, 0.17 mol) was slowly added a 1M CH₂Cl₂ solution of BCl₃ (260 mL, 0.26 mol) over 30 minutes at zero degrees C. The mixture was stirred 10 hours at room temperature. Aqueous HCl (1N, 250 mL) was added very slowly, followed by the addition of H₂O (500 mL) and the aqueous layer was extracted with CH₂Cl₂ (3 x 500 mL). The combined organic layers were washed with brine (200 mL) and dried over MgSO₄. After evaporation of the solvent, the crude product was crystallized from ethyl acetate to give 37.4 g of Compound **6**. The mother liquors were concentrated and purified by flash SiO₂ chromatography to give 9.1 g of Compound **6** (46.5 g, 93 percent overall) as white needles; m.p. 128-130 degrees C from ethyl acetate.

¹H NMR (250 MHz, CDCl₃) δ 10.09 (s, 1H, CHO); 9.66 (s, 1H, OH); 6.52 (s, 1H, CH arom.); 4.02 (s, 3H, OCH₃); 3.97 (s, 3H, OCH₃). IR (film) 3085, 2960, 1712, 1633, 1204, 1026, 834 cm⁻¹. EIMS m/z 288 (M⁺). EIHRMS calcd for C₁₀H₉BrO₅ (M⁺) 287.9633, found 287.9622. Anal. calcd. for C₁₀H₉BrO₅: C, 41.53; H, 3.14; Br, 27.65. Found: C, 41.66; H, 3.10; Br, 27.35.

### Example 4: Compound 8

To a THF solution (240 mL) of Compound **6** (10.43 g, 36.0 mmol) was added NaH (97 percent, 912 mg, 37.0 mmol) at -10 degrees C, and the mixture was stirred at -5 degrees C to zero degrees C for 15 minutes to give a yellow clear solution. Diisobutylaluminum hydride (DIBAL; 120 mL, 1.5M solution in toluene, 180 mmol) was added over 10 minutes to the above mixture at -5 degrees C to zero degrees C and the reaction was stirred at this temperature for one hour (h). The solution was cooled to -20 degrees C and was carefully quenched with methanol (MeOH) (6 mL) followed by aqueous HCl (1N, 50 mL) and THF-H₂O (4:1, 250 mL).

NaIO₄ (20.6 g, 96.3 mmol) was added at room temperature followed by the addition of aqueous HCl (1N, 50 mL). After 30 minutes additional aqueous HCl (1N, 50 mL) was added and the reaction was stirred for 3 hours. The mixture was filtered through celite, washing the celite pad with THF-H₂O (4:1, 200 mL) and ethyl acetate (500 mL) and the layers were separated. The aqueous layer was extracted with ethyl acetate (2 x 100 mL), the combined organic layers were washed with saturated NaHCO₃ solution (300 mL), followed by brine (150 mL) and dried over MgSO₄. Solvents were evaporated (without heat) an the crude yellow solid was used for the next reaction without any further purification.

A sample of the crude Compound **8** was purified by flash SiO₂ chromatography (1:1 hexanes/ethylacetate) to give a pure Compound **8** as a white solid; m.p. 47-49 degrees C from ethyl acetate.

¹H NMR (250 MHz, CDCl₃) δ 5.72 (s, 1H, =CH-); 4.29 (br. s, 2H, CH₂-O); 3.90 (s, 3H, OCH₃); 3.82 (s, 1H, OH); 3.37 (ABq, 2H, J_{ab} = 7.6 Hz, Δv - 47.6 Hz, CH₂-spiroepoxide). IR (CDCl₃) 3593, 3376, 2943, 1656, 1561, 1365, 1250, 898, 742 cm⁻¹ . EIMS m/z, 260 (M⁺). EIHRMS calcd. for C₉H₉BrO₄ (M⁺) 259.9683, found 259.9686.

### Example 5: Compound 9

Dess-Martin periodinane [Dess et al., J. Org., Chem. 48:4155 (1983); 18.3 g, 43.2 mmol] was added under nitrogen to a solution of the above Compound **8** in dry CH₂Cl₂ at zero degrees C. The reaction was stirred for one hour at zero degrees C and then for one hour at room temperature. The mixture was treated with K₂CO₃ (40 g) and H₂O (3-5 mL) filtered through celite three times and finally filtered through a plug of SiO₂ (washing with ethyl acetate). The clear yellow solution obtained was concentrated and the crude product was purified by flash SiO₂ chromatography (5:3:2 hexanes/ethyl acetate/methylene chloride) to give 3.70 g of Compound **9** (40 percent overall yield from Compound **8**) as a yellow solid; m.p. 134-136 degrees C from CH₂Cl₂.

¹H NMR (250 MHz, CDCl₃) δ 9.96 (s, 1H, CHO); 5.89 (s, 1H, = CH); 3.95 (s, 3H, OCH₃); 3.68 (ABq, 2H, J_{ab} = 8.7 Hz, Δv = 192.5 Hz, spiroepoxide-CH₂). IR (CDCl₃) 1692, 1654, 1603, 1327, 1251, 1226 cm⁻¹. EIMS. m/z 258 (M⁺). EIHRMS calcd (M⁺) 257.9527, found 257.9515. Anal. calcd for C₉H₇BrO₄: C, 41.71; H, 2.72; Br, 30.86. Found: C, 41.53; H, 2.77; Br, 30.87

### Example 6: Compound 11

A solution of 1,6-bis-TMS-hexenediyne (6.00 g, 27.2 mmol) in THF-H₂O (3:1, 100 mL) at 20 degrees C was treated with LiOH·H₂O (5.90 g, 140 mmol). The reaction was stirred 2 hours at 20 degrees C, diluted with pentane (200 mL), washed with brine (2 x 100 mL), dried over MgSO₄ and filtered through CaSO₄, all under nitrogen as much as possible. The filtrate was evaporated to 60 mL under a stream of nitrogen. 1,10-Phenanthroline (10-15 mg) was added under nitrogen and the solution was cooled to zero degrees C.
n-Butyllithium (2.5M solution in hexane) was added until indicator change occurred, then 14.8 mL (37.0 mmol) more was added, giving a brown purple solution of 1,6-dilithiohex-3-ene-1,5-diyne (Compound **10**). The mixture was cooled to -78 degrees C.

n-Butyllithium (3.60 mL, 2.5M hexane solution, 9.00 mmol) was added under nitrogen to a solution of N-methylaniline (1.00 mL, 9.20 mmol) in dry THF (20 mL) at zero degrees C. The mixture was cooled to -78 degrees C and added via cannula to a slurry of aldehyde Compound **9** (2.06 g, 7.95 mmol) in dry THF (50 mL) at -78 degrees C. The cold bath was removed and the reaction was allowed to warm until the aldehyde went into solution completely (yellow suspension becomes orange solution). The reaction mixture was recooled to -78 degrees C and treated with the above solution of dilithioenediyne (Compound **10**) via cannula. The mixture was stirred one hour at -78 degrees C and 15 minutes at -43 degrees C, and was then quenched with saturated NH₄Cl solution (30 mL) and diluted with ether (600 mL). The organic layer was washed consecutively with aqueous HCl (1N, 100 mL), aqueous HCl (0.1N, 100 mL), saturated NaHCO₃ solution (100 mL) and brine (100 mL), then dried over MgSO₄. Solvents were evaporated and the crude product was subjected to flash SiO₂ chromatography (4:1 hexanes/ethyl acetate) to give 2.14 g of Compound **11** (80 percent) as a brown oil.

¹H NMR (250 MHz, CDCl₃) δ 10.02 (s, 1H, CHO); 5.92 (m, 2H, CH=CH); 5.37 (s, 1H, CH enol ether); 3.76 (s, 3H, MeO); 3.67 (ABq, 2H, J_{ab}= 5.4 Hz, Δv = 59.5 Hz, spiroepoxide-CH2); 3.40 (d, 1H, J = 1.4 Hz, =CH); 2.37 (s, 1H, OH).IR (CDCl₃) 3572, 3303, 1684, 1622, 1342, 1247 cm^{1.}

### Example 7: Compound 12

A solution of Compound **11** (2.14 g, 6.39 mmol) in dry CH₂Cl₂ (100 mL) was treated under nitrogen with triethylamine (Et₃N) (6.00 mL, 43.0 mmol) and trimethylsilyl trifluoroacetate (3.60 mL, 20.8 mmol) at zero degrees C for 30 minutes and then 1.5 hour at room temperature. The reaction was quenched by the addition of saturated NH₄Cl solution (30 mL) and diluted with ether (600 mL). The organic layer was washed with aqueous HCl (0.1N, 30 mL), saturated NaHCO₃ solution (30 mL), brine (30 mL) and dried over MgSO₄. The solvents were evaporated and the crude product was purified by flash SiO₂ chromatography (10:1 hexanes/ethyl acetate) to afford 1.95 g of Compound **12** (75 percent) as a yellow oil.

¹H NMR (250 MHz, CDCl₃) δ 10.04 (s, 1H, CHO); 5.96-5.87 (m, 2H, CH=CH); 5.37 (s, 1H, CH enol ether); 3.75 (br. s, 3H, OCH₃); 3.72 (ABq, 2H, J_{ab} = 5.4 Hz, Δv = 116.5 Hz, spiroepoxide-CH₂); 3.37 (d, 1H, J = 1.8 Hz, =CH); 0.18 (br. s, 9H, Me₃Si-). IR (CHCl₃) 3302, 2962, 1681, 1267, 1250, 1046 cm⁻¹. EIMS m/z 406 (M⁺).

### Example 8: Compound 13

Potassium hexamethyldisilazide (7.20 mL, 1.4M solution in THF, 10.0 mmol) was added under nitrogen to a solution of 3-ethyl-3-pentanol (1.80 mL, 12.8 mmol) in dry toluene (200 mL) at zero degrees C. After 10 minutes, the mixture was cooled to -78 degrees C. A solution of Compound **12** (2.00 g, 5.97 mmol) in dry toluene (30 mL) at zero degrees C was added to the above mixture via syringe affording a yellow-orange solution. After 20 minutes at -78 degrees C, the reaction was quenched by the addition of pH 7 buffer solution (50 mL) followed by ethyl acetate (300 mL). The aqueous layer was extracted with ethyl acetate (4 x 100 mL); the combined extracts were dried over MgSO₄. The solvents were evaporated and the crude product was purified by flash SiO₂ chromatography (8:1 hexanes/ethyl acetate) to give 1.10 g of Compound **13** (55 percent) as a white foam.

¹H NMR (250 MHz, CDCl₃) δ 6.10 (d, 1H, J = 9.5 Hz, CH=CH); 5.99 (dd, 1H, J = 9.5, 1.7 Hz, CH=CH); 5.83 (dd, 1H, J = 11.8, 1.7 Hz, CH proparg.); 4.84 (s, 1H, = CH-enol ether); 3.75 (d, 1H, J = 11.8 Hz, OH); 3.67 (s, 3H, OCH₃); 3.15 (ABq, 2H, J_{ab} = 5.8 Hz, Δv = 148.2 Hz, spiroepoxide-CH₂); 0.26 (br. s, 9H, Si(CH₃)₃). IR (film) 3508, 2956, 1621, 1237, 1122, 846, 738 cm⁻¹ . EIMS m/z 406 (M⁺). CIHRMS calcd for C₁₈H₂₀BrSiO₄ (M+H) 407.0314, found 407.0307. Anal. Calcd. for C₁₈H₁₉BrSiO₄: C, 53.20; H, 4.72. Found. C, 52.84; H, 4.55.

### Example 9: Compound 14

A solution of the bicycle Compound **13** (2.24 g, 5.50 mmol) in 38.0 mL of ethylene glycol and 10.0 mL of dry THF, was treated under nitrogen with camphorsulfonic acid (CSA; 48.0 mg, 0.21 mmol). After stirring at 50 degrees C for 45 minutes the mixture was cooled and quenched with pyridine (1 mL). The solution was diluted with ethyl acetate (800 mL) and washed with H₂O (2 x 75 mL) and brine (75 mL). The organic layer was dried over MgSO₄, filtered and concentrated in vacuo to a volume of approximately 150 mL. The latter was filtered through a plug of SiO₂ (6:4 hexanes/ethyl acetate). The filtrate was concentrated to give 1.78 g of ketal Compound **14** (89 percent) as a white amorphous solid; m.p. >180°C δ.

¹H-NMR (250 MHz, CDCl₃) δ 5.97-5.87 (m, 2H, CH=CH); 5.76 (dd, 1H, J = 11.8, 1.3 Hz, CH proparg.); 4.33-4.01 (m, 4H, ethylene ketal); 3.62 (d, 1H, J = 11.8 Hz, OH proparg.); 3.33 (ABq. 2H, J = 5.1 Hz, Δv = 80.8 Hz, spiroepoxide-CH₂); 2.48 (ABq, 2H, J = 13.6 Hz, Δv = 83.6 Hz, CH₂ in six membered ring); 2.47 (s, 1H, OH). IR (film) 3379, 2897, 1169, 1093, 1028, 735 cm⁻¹. CIHRMS calcd for C₁₆H₁₄BrO₅ (M+H) 365.0024, found 365.0040.

### Example 10: Compound 15

A solution of the ketal Compound **14** (1.50 g, 4.11 mmol) in anhydrous DMSO (40 mL) was treated under nitrogen with potassium acetate (KOAc) (1.30 g, 13.1 mmol) and acetic acid (AcOH) (0.85 mL, 14.8 mmol). After stirring the reaction at 55 degrees C for 3 hours, the mixture was cooled and poured into a saturated aqueous NaHCO₃ (100 mL). The mixture was extracted with ethyl acetate (2 x 500 mL) and the combined organic layers were dried over MgSO₄, filtered and concentrated in vacuo to a volume of approximately 150 mL. The residue was filtered through a plug of SiO₂ (1:1 hexanes/ethyl acetate) and the solvents were evaporated in vacuo to afford 1.54 g (88 percent) of Compound **15** as a colorless glass.

¹H NMR (250 MHz, CDCl₃) δ 5.97-5.92 (m, 2H, CH=CH); 5.83 (dd, 1H, J = 9.6, 1.6 Hz, CH proparg.); 4.60 (s, 1H, OH); 4.46 (ABq, 2H, J = 12 Hz, Δv = 26.3 Hz, CH₂-OAc); 4.30 (d, 1H, J = 9.6 Hz, OH proparg.); 4.27-3.96 (m, 4H, ethylene ketal); 3.47 (s, 1H, OH); 2.53 (ABq, 2H, J = 14.1 Hz, Δv = 24.9 Hz, CH₂ six membered ring); 2.12 (s, 3H, OCH₃). IR (CDCl₃) 3490, 2961, 2897, 1735, 1236, 1161, 1039 cm⁻¹. EIMS m/z 426 (M+H). CIHRMS calcd. for C₁₈H₁₈BrO₇ (M+H) 425.0235, found 425.0215.

### Example 11: Compound 16

A saturated solution of NH₃ in methanol (40 mL) was added to the crude Compound **15** (1.54 g, 3.60 mmol) at room temperature. After stirring the reaction mixture for 1 hour, the volatiles were removed in vacuo and the residue was dissolved in acetone/pH 7 phosphate buffer (1:1, 40 mL) at zero degrees C. NaIO₄ (12.0 mL, 0.3 M solution in H₂O, 3.60 mmol) was added and the reaction was stirred at zero degrees C for 30 minutes. The mixture was diluted with ethyl acetate (50 mL) and filtered through a plug of SiO₂(washed with ethyl acetate). The aqueous layer was extracted with ethyl acetate (4 x 25 mL). The combined organics layers were dried over MgSO₄, filtered and concentrated to give 1.00 g of enone Compound **16** (80 percent) as a yellow amorphous solid; m.p. >130 degrees C d.

¹H NMR (250 MHz, CDCl₃) δ 5.96-5.84 (m, 3H, CH=CH and CH proparg); 4.56 (d, 1H, J= 11.4 Hz, OH proparg.); 4.41-4.08 (m, 4H, ethylene ketal); 3.77 (d, 1H, J= 8.0 Hz, OH); 2.62 (ABq, 2H, J_{ab} = 14.0 Hz, Δv = 94.0 Hz, CH₂ six membered ring). IR (film) 3378, 2899, 1702, 1168, 1114, 1099 cm⁻¹. EIMS m/z 352 (M+H). CIHRMS calcd. for C₁₅H₁₂BrO₅ (M+H) 350.9868, found 350.9889. Anal. calcd. for C₁₅H₁₁BrO₅: C, 51.29; H, 3.16; Br, 22.76. Found: C, 51.54; H, 3.44; Br, 22.79.

### Example 12: Compound 17

A solution of the enone Compound **16** (1,16 g, 3.30 mmol) in MeOH (115 mL) and H₂O (4 mL) was treated with NaN₃ (539 mg, 8.28 mmol). After stirring at 55 degree C (bath temperature) for 4 hours, the mixture was cooled and diluted with ethyl acetate (50 mL). The solution was filtered through a plug of SiO₂, washing with ethyl acetate. The solvents were evaporated and the residue was subjected to a SiO₂ chromatography (1:1 hexanes/acetone) to afford 0.84 g (82 percent) of Compound **17** as a yellow solid; m.p. >118 degrees C d.

¹H NMR (250 MHz, CDCl₃) δ 5.94-5.85 (m, 2H, CH=CH); 5.73 (dd, 1H, J= 11.3, 1.0 Hz, CH proparg.); 4.60 (d, 1H, J= 11.3 Hz, OH proparg.); 4.36-4.15 (m, 4H, ethylene ketal); 3.89 (br. s, 1H, OH); 2.51 (ABq, 2H, J_{ab}= 13.8 Hz, Δv = 129.1 Hz, CH₂ in six membered ring); IR (CDCl₃) 3508, 2908, 2126 (int), 1678, 1610, 1343, 1314, 1111, 1031 cm⁻¹.

### Example 13: Compound 18

A solution of diethyl phosphonoacetic acid [Cook et al., Synthesis, 283 (1981) (2.50 g, 12.8 mmol)] in 47 mL of dry benzene was treated under nitrogen at room temperature with oxalyl chloride (3.40 mL, 38.4 mmol) followed by dimethylformamide (DMF) (0.21 mL). After stirring one hour, the volatiles were removed under a stream of nitrogen and then in vacuo. The residue was dissolved in dry THF (30 mL) giving a 0.43M solution of acid chloride.

Pyridine (0.79 mL, 9.78 mmol) was added under nitrogen to a solution of alcohol Compound **17** (1.02 g, 3.26 mmol) in dry THF at zero degrees C. The above acid chloride (11.4 mL, 4.89 mmol) was added and the reaction mixture was stirred 15 minutes at zero degrees C. Ethyl acetate was added and the solution was filtered through a plug of SiO₂, washing with ethyl acetate. Solvents were evaporated and the residue was purified by a flash SiO₂ chromatography (ethyl acetate) to give 1.02 g (64 percent) of Compound **18** as a white solid; m.p. 109-11 degrees C from ethyl acetate.

¹H NMR (250 MHz, CDCl₃) δ 6.60 (d, 1H, J = 1.5 Hz, CH proparg.); 5.97 (d, 1H, J = 9.5 Hz, CH=CH); 5.86 (dd, 1H, J = 9.5, 1.5 Hz, CH=CH); 4.36-4.08 (m, 8H, ethylene ketal; (O-CH₂- Me)₂); 3.11 (ABq, 2H, J_{ab}= 9.3 Hz, Δv = 21.1 Hz, CH₂-P(O)-); 2.48 (ABq, 2H, J_{ab} = 13.8 Hz, Δv = 141.2 Hz, CH₂ six membered ring); 1.35 (t, 6H, J = 7.0 Hz, (OEt)₂). IR (film) 3270, 2987, 2134, 1746, 1700, 1609, 1339, 1254, 1109, 1024 cm⁻¹. HRMS (FAB-NOBA) calcd. for C₂₁H₂₃PN₃O₉ (M+H) 492.1165, found 492.1173.

### Example 14: Compound 19

A solution of Compound **18** (0.83 g, 1.70 mmol) in dry THF (85 mL) was cooled at zero degrees C under nitrogen and treated with LiBr (473 mg, 5.44 mmol) followed by Et₃N (2.37 mL, 17.0 mmol). After stirring at zero degrees C for 15 minutes, the reaction mixture was allowed to stir at room temperature for 4 hours. The solution was filtered through a plug of SiO₂, washing with ethyl acetate. The filtrate was concentrated and the residue was chromatographed (1:1 hexanes/acetone) to give 527 mg (92 percent) of Compound **19** as a yellow solid; m.p. 113°C d.

¹H NMR (250 MHz, CDCl₃) δ 6.13 (s, 1H, =CH enone); 6.09 (d, 1H, J = 1.5 Hz, CH proparg.); 5.93 (d, 1H, J = 9.5 Hz, CH=CH); 5.85 (dd, 1H, J = 9.5, 1.5 Hz, CH=CH); 4.35-4.15 (m, 4H, ethylene ketal); 2.78 (br s, 1H, OH); 2.44 (ABq, 2H, J_{ab}= 13.3 Hz, Δv = 77.36 Hz, CH₂ six membered ring). IR (film) 3358, 2986, 2126, 1713, 1645, 1340, 1022 cm⁻¹. HRMS (FAB-NOBA) calcd. for C₁₇H₁₂N₃O₅ (M+H) 338.0777, found 338.0783.

### Example 15: Compound 20

Hydrogen sulfide was bubbled into a stirred solution of lactone Compound **19** (0.68 g, 2.02 mmol) in MeOH (90 mL) containing piperidine (0.3 mL) over 20 minutes while the temperature was maintained at zero degrees C. Ethyl acetate (50 mL) and H₂O (50 mL) were then added carefully and the mixture was extracted with ethyl acetate (5 x 20 mL). The combined organic layers were washed with brine (2 x 15 mL), dried over MgSO₄ and concentrated in vacuo. The residue was flash chromatographed (1:1 hexanes/acetone) to give 534 mg (85 percent) of compound **20** as a yellow solid; m.p. >70 degrees C d.

¹H NMR (250 MHz, Acetone-d₆) δ 6.14 (d, 1H, J = 1.5 Hz, cH proparg.); 5.97 (d, 1H, J = 9.5 Hz, CH=CH); 5.83 (dd, 1H, J = 9.5, 1.5 Hz, CH=CH); 5.76 (s, 1H, =CH enone), 5.63 (br s, 1H, NH); 5.49 (br. s, 1H, NH); 4.16-4.02 (m, 4H, ethylene ketal); 2.80 (br. s, 1H, OH); 2.33 (ABq, 2H, J_{ab}= 13.4 Hz, Δv = 88.8 Hz, CH₂ six membered ring). IR (film) 3360 (br.), 1642, 1609, 1409, 1177, 1011 cm⁻¹. EIMS m/z 311 (M⁺). CIHRMS calcd for C₁₇H₁₄NO₅ (M+H) 312.0872, found 312.0883.

### Example 16: Compound 22

Trichloromethyl chloroformate (0.174 mL, 1.45 mmol) was added under nitrogen to a solution of vinyl amine Compound **20** (180 mg, 0.58 mmol) in dry CH₂Cl₂ (40 mL) followed by pyridine (0.70 mL, 8.7 mmol). After stirring 4 hours at room temperature, the reaction mixture was cooled to zero degrees C. Pyridine (0.70 mL, 8.7 mmol) was added, followed by MeOH (5 mL). After 30 minutes at zero degrees C, the reaction was quenched by the addition of H₂O (10 mL) followed by ethyl acetate/ether (1:1, 20 mL). The aqueous layers were extracted with ethyl acetate (4 x 20 mL). The combined organic layer was dried over MgSO₄, filtered and concentrated in vacuo. The residue was subjected to a flash SiO₂ chromatography (1:2 hexanes/ethyl acetate) to give 125 mg (50 percent) of Compound **22** as a yellow glass.

¹H NMR (250 MHz, CDCl₃) δ 6.28 (s, 1H, =CH enelactone); 6.18 (d, 1H, J = 1.5 Hz, CH proparg.); 6.04 (br. s, 1H, NH); 5.96 (d, 1H, J = 9.6 Hz, CH=CH); 5.89 (dd, 1H, J = 9.6, 1.5 Hz, CH=CH); 4.23-4.14 (m, 1H, ethylene ketal); 4.12-3.97 (m, 3H, ethylene ketal); 3.87 (s, 3H, -OCH₃ carbonate); 3.79 (s, 3H, - OCH₃ carbamate); 2.75 (ABq, 2H, J_{ab} = 13.4 Hz, Δv = 224.6 Hz, CH₂ in six membered ring). IR (CDCl₃) 3404, 2977, 2872, 1733 (br), 1275 cm⁻¹. CIHRMS calcd for C₂₁H₁₈NO₉(M+1) 428.0981, found 428.0983. Anal. calcd. for C₂₁H₁₈NO₉: C, 59.00; H, 4.01; N, 3.28. Found: C, 58.60; H, 4.40; N, 2.86.

### Example 17: Compound 23a

Diisobutylaluminum hydride (1.60 mL, 1.5 M solution in toluene, 2.4 mmol) was added under nitrogen to a solution of Compound **22** (170 mg, 0.40 mmol) in anhydrous CH₂Cl₂(40 mL) at -78 degrees C. After 15 minutes the reaction was quenched by the addition of MeOH (2 mL) at -78 degrees C. The cold bath was removed and the reaction mixture was allowed to warm to room temperature. The solution was diluted with ethyl acetate (15 mL). A saturated solution of potassium sodium tartrate (Rochelle's salt) (5 mL) was added and the mixture was stirred until the two phases were clear (2 hours). The aqueous layer was further extracted with ethyl acetate (3 x 15 mL) and combined organics were dried over MgSO₄. Purification of the crude product by 5 cm SiO₂ flash chromatography (1:1 hexanes/acetone) afforded 116 mg (78 percent) of lactol Compound **23a** as a colorless glass.

¹H NMR(250 MHz, Acetone-d₆) δ 6.01-5.76 (m, 3H); 5.65-5.27 (m, 4H); 4.15-3.86 (m, 4H, ethylene ketal); 3.64 (s, 3H, OCH₃); 2.44 (1/2ABX, 1H, J_{ab}= 13.4 Hz, Jₐₓ = 3.6 Hz, CH₂ six membered ring); 2.12 (1/2ABX, 1H, J_{ab} = 13.4 Hz, J_{bx} = 5.4 Hz,CH₂ six membered ring). IR (film) 3335, 1708, 1702, 1503, 1252, 1034 cm⁻¹.

### Example 18: Compound 23

NaBH₄ (64.0 mg. 1.68 mmol) was added to a solution of lactol Compound **23a** (39.0 mg, 0.105 mmol) in anhydrous MeOH (3.20 mL) and H₂O (4 drops) at zero degrees C. After one hour, the reaction was quenched by the addition of AcOH (1 mL) and H₂O (4 drops) at zero degrees C. The mixture was stirred for 5 minutes and concentrated in vacuo. THF (1 mL), MeOH (1.8 mL) and H₂O (2 drops) were added to the mixture and it was allowed to stir for 5 minutes. The solvents were evaporated and THF (5 mL), followed by MeOH (5 drops) and H₂O (5 drops) was added to the residue. The resulting solution was stirred for 5 minutes. This operation was repeated five times, adding celite to make the solid more consistent. After the last repetition the mixture was filtered through plug of SiO₂ and washed with THF (5 x 2 mL). Solvents were evaporated and the residue was subjected to SiO₂ flash chromatography (ethyl acetate first and then 95:5 ethyl acetate/methanol) to give 25.5 mg (65 percent) of triol Compound **23** as a white powder.

¹H NMR (250 MHz, CDCl₃) δ 6.72 (br. s, 1H, NH); 6.42 (dd, 1H, J = 8.0, 7.0 Hz, vinyl CH); 5.84 (br. s, 2H, CH = CH); 5.61 (br. s, 1H, CH proparg.); 4.74 (br. s, 1H, OH); 4.37-4.18 (m, 2H, 1H from allylic CH₂ and 1H from ethylene ketal); 4.11-3.94 (m, 4H, 1H from allylic CH₂ and 3H from ethylene ketal); 3.80 (s, 3H, OCH₃); 3.27 (br. s, 1H, OH); 2.72 (br. s, 1H, OH); 2.46 (ABq, 2H, J_{ab} = 14.3 Hz, Δv = 63.8 Hz, CH₂ six membered ring); IR (film) 3313, 2924, 1704, 1503, 1242, 1020 cm⁻¹.

### Example 19: Compound 24

Diisopropylazodicarboxylate (0.067 mL, 0.34 mmol) was added under nitrogen to a solution of triphenylphosphine (88.0 mg, 0.34 mmol) in dry THF (3 mL) at zero degrees C. After stirring 30 minutes at zero degrees C, a slurry yellow solution had formed. Thiolacetic acid (0.048 mL, 0.67 mmol) was added to the above solution followed by triol Compound **23** (25 mg, 0.067 mmol) in dry THF (2 mL). After stirring 20 minutes at zero degrees C the reaction was quenched with saturated aqueous NaHCO₃ (3 mL). The mixture was diluted with ethyl acetate (5 mL) and extracted with ethyl acetate (3 x 4 mL). The organic phase was dried over MgSO₄, filtered and concentrated. Purification of the crude product by SiO₂ flash chromatography (4:6 hexanes/ethyl acetate) afforded 12.5 mg (43%) of thiolacetate Compound **24**.

¹H NMR (250 MHz, CDCl₃) δ 6.75 (br. s, 1H, NH); 6.21 (t, 1H, J = 8.3 Hz, vinyl CH ); 5.83 (dd, 2H, J = 17.0, 9.7 Hz, CH = CH); 5.59 (d, 1H, J = 3.8 Hz, CH proparg.); 4.45 (br. s, 1H, OH); 4.19-3.94 (m, 6H, CH₂-S and ethylene ketal); 3.79 (s, 3H, OCH₃); 2.44 (ABq, 2H, J_{ab} = 14.3 Hz, Δv = 55.0 Hz, CH₂ six membered ring); 2.48 (br. s, 1H, OH); 2.32 (s, 3H, C(O)CH₃). IR (CDCl₃) 3392, 2961, 2250, 1729, 1681, 1502, 1227 cm⁻¹.

### Example 20: Compound 25

Diisobutylaluminum hydride (0.28 mL, 1M solution in cyclohexane, 0.28 mmol) was added under nitrogen to a solution of thiolacetate Compound **24** (12.0 mg, 0.028 mmol) in anhydrous CH₂Cl₂ (5 mL) at -78 degrees C. After 30 minutes the reaction was quenched by the addition of MeOH (5 drops) at -78 degrees C. The cold bath was removed and the reaction mixture was allowed to warm to room temperature. The solution was diluted with ethyl acetate (10 mL) and saturated aqueous potassium sodium tartrate (Rochelle's salt, 3 mL) was added. The mixture was stirred 30 minutes until the two phases were clear. The layers were separated and the aqueous layer was further extracted with ethyl acetate (3 x 5 mL), dried over MgSO₄ and concentrated.

Phthalimido methyl disulfide [Magnus et al., J. Chem. Soc., Chem. Commun., 916 (1989) (30 mg, 0.13 mmol)] was added to a solution of the above residue in dry CH₂Cl₂ (1.5 mL) and dry THF (3 drops) at room temperature. After stirring 30 minutes the reaction mixture was subjected to SiO₂ flash chromatography (1:1 hexanes/acetone) to afford 6 mg (46 percent) of trisulfide Compound **25** as a colorless glass.

¹H NMR (250 MHz, CDCl₃) δ 6.81 (br. s, 1H, NH); 6.46 (dd, 1H, J = 9.5, 6.1 Hz, vinyl CH ); 5.83 (dd, 2H, J = 14.6, 9.6 Hz, CH = CH); 5.57 (s, 1H, CH proparg); 4.39 (dd, 1H, J = 14.2, 9.5 Hz, CH₂-S-); 4.12-3.81 (m, 5H, 1H from CH₂-S- and 4H from ethylene ketal); 3.80 (s, 3H, carbamate OCH3); 2.56 (s, 3H, S- CH₃); 2.48 (ABq, 2H, J_{ab} = 14.3 Hz, Δv = 46.0 Hz, CH₂ six membered ring). IR (CDCl₃) 3396, 2923, 1736, 1502, 1235 cm⁻¹. HRMS(FAB-GLY) calcd. for C₂₀H₂₂NO₆S₃ (M+H) 468.0611, found 468.0606.

### Example 21: Compound 2 (Calicheamicinone)

Camphorsulphonic acid (2.0 mg , 0.009 mmol) was added to a solution of trisulfide Compound **25** (5.0 mg, 0.011 mmol) in THF (1 mL) and H₂O (3 drops) at room temperature. After stirring 8 hours, the reaction mixture was diluted with hexanes (1 mL) and subjected to SiO₂ flash chromatography (6:4 hexanes/ethyl acetate) to give 3 mg (65 percent) of calicheamicinone Compound **2** as a white solid.

¹H NMR (490 MHz, CDCl₃) δ 6.93 (br. s, 1H, NH); 6.48 (dd, 1H, J = 9.3. 6.4 Hz, vinyl CH ); 6.03 (d, 1H, J = 5.4 Hz, CH proparg.); 5.90 (dd, 2H, J = 11.0, 9.3 Hz, CH = CH); 4.12 (dd, 1H, J = 14.0, 9.3 Hz, CH₂-S); 3.87 (dd, 1H, J = 14.0, 6.4 Hz, CH₂-S); 3.79 (s, 3H, carbamate OCH3); 3.21 (s, 1H, OH); 3.03 (ABq, 2H, J_{ab} = 17.0 Hz, Δv = 176.6 Hz, CH₂ six membered ring); 2.64 (s, 1H, OH); 2.55 (s, 3H, S-CH₃). IR (CDCl₃) 3582, 3374, 2925, 1732, 1678, 1497, 1236 cm⁻¹. HRMS (FAB-NOBA) calcd. for C₁₈H₁₈NO₅S₃ (M+H) 424.0348, found 424.0359.

### Example 22: Preparation of Methyl Phtalimido Disulfide

Preparation of this reagent was based on the general procedure of Sullivan et al., J. Sulfur Chem. A, 1:207 (1971). A suspension of phthalimide (7.36 g, 0.050 mol) and triethylamine (Et₃N) (6.97 mL, 0.050 mol) in 120 mL CH₂Cl₂ was added over 40 minutes to a vigorously stirred solution of SCl₂ (5.15 g, 0.050 mol) in 25 mL CH₂Cl₂ at zero degrees C. Methanethiol (2.78 mL, 0.050 mol, condensed at -78°C) was added to Et₃N (6.97 mL, 0.050 mol) in 10 mL CH₂Cl₂ at zero degrees C, and this solution was added over 12 minutes to the above reaction mixture at zero degrees C. The resulting mixture was stirred for 8 hours at zero degrees C and 40 minutes at room temperature, then washed successively with H₂O (40 mL), 5 percent Na₂CO₃ solution (20 mL), and H₂O (20 mL), dried over Na₂SO₄ and concentrated in vacuo. The derived solid was extracted with hot benzene (80 mL) and filtered, washing with benzene (remaining solid was discarded).

The filtrate was then concentrated and the residue dissolved in a minimal amount of hot ethanol. After this solution had been cooled to -23°C, the slightly yellow precipitate was collected by filtration and washed successively with ethanol, water and ethanol. This procedure provided 3.05 g of crude product which could be conveniently purified by flash column chromatography (hexanes/Et₂O/CH₂Cl₂, 60:35:5) to deliver 2.10 g (19 percent) of the disulfide as a colorless solid. The reagent was so employed in subsequent transformations; a small portion was crystallized from CH₂Cl₂/ethanol to obtain needles: mp. 157-9°C; IR (CHCl₃) 3020, 1785, 1740, 1705, 1340, 1275, 1055, 870 cm^{-1; 1}H NMR, (250 MHz, CDCl₃) δ 7.95 (dd, J = 3.1, 5.5 Hz, 2H), 7.80 (dd, J = 3.0, 5.5 Hz, 2H), 2.77 (s, 3H); chemical ionization mass spectrum, 226.0015 (M+H calcd for C₉H₈NO₂S₂, 225.9997).

### Analytical Data for Compounds 28-33 and 35

### Compound 28

A slightly yellow glass: IR (CH₂Cl₂) 3560, 3610-3180, 3045, 2970, 2880, 1330, 1150, 1110, 1070, 1050, 1015, 950 cm⁻¹; ¹H NMR (250 MHz, CDCl₃) δ 6.17 (app t, J = 8.2 Hz, 1H), 5.89 (d, J = 9.5 Hz, 1H), 5.82 (dd, J = 1.4, 9.5 Hz, 1H), 5.82 (s, 1H), 5.36 (d, J = 7.8 Hz, 1H), 4.06-3.89 (m, 4H), 3.62-3.50 (m, 1H), 3.46-3.35 (m, 1H), 2.73 (d, J = 8.0 Hz, 1H), 2.41 (ABq, J = 13.7, Δv = 92.4 Hz, 2H), 2.39 (s, 1H), 1.67 (dd, J = 6.7, 7.3 Hz, 1H); chemical ionization mass spectrum, 317. 0857 (M+H calcd for C₁₇H₁₇O₄S, 317.0848).

### Compound 29

A colorless glass: IR (CHCl₃) 3610-3160, 3580, 3010, 1330, 1150, 1075, 1050, 1020, 950 cm⁻¹; ¹H NMR (250 MHz, CDCl₃) δ 6.26 (app t, J = 7.9 Hz, 1H), 5.90 (d, J = 9.5 Hz, 1H), 5.82 (dd, J = 1.3, 9.4 Hz, 1H), 5.82 (s, 1H), 5.37 (d, J = 7.8 Hz, 1H), 4.07-3.82 (m, 6H), 2.64 (d, J = 7.9 Hz, 1H), 2.64-2.57 (m, 1H); 2.57 (s, 3H), 2.44 (s, 1H), 2.27 (d, J = 13.6 Hz, 1H). FAB mass spectrum, 395.0442 (M+H calcd for C₁₈H₁₉O₄S₃, 395.0446).

### Compound 30

A colorless glass: IR (CHCl₃ 3610-3150, 3580, 3010, 1335, 1150, 1075, 1050 cm⁻¹; ¹H NMR (250 MHz, CDCl₃) δ 7.34-7.28 (m, 5H), 6.24 (app t, J = 7.8 Hz, 1H), 5.89 (d, J = 9.5 Hz, 1H), 5.81 (dd, J = 1.4, 9.5 Hz, 1H). 5.80 (s, 1H), 5.35 (d, J = 7.8 Hz, 1H), 4.11 (app s, 2H), 4.09-3.75 (m, 6H), 2.62-2.56 (m, 2H), 2.39 (s, 1H), 2.26 (d, J = 13.4 Hz, 1H); FAB mass spectrum, 471.0779 (M+H calcd for C₂₄H₂₃O₄S₃, 471.0760).

### Compound 31

A colorless glass: IR (CHCl₃) 3610-3150, 3580, 3020, 1665, 1125, 1050 cm⁻¹; ¹H NMR (250 MHz, CDCl₃) δ 6.47-6.40 (m, 1H), 6.12 (s, 1H), 5.92 (d, J = 9.5 Hz, 1H), 5.86 (dd, J = 1.4, 9.5 Hz, 1H), 5.52 (d, J = 7.9 Hz, 1H), 3.95 (1/2 ABX, J_{AB} = 13.6, J_{AX} = 8.8 Hz, 1H), 3.85 (1/2 ABX, J_{BA} = 13.6, J_{BX} = 7.5 Hz, 1H), 3.10 (dd, J = 1.0, 17.0 Hz, 1H), 2.74 (d, J = 16.9 Hz, 1H), 2.68 (s, 1H), 2.66 (d, J = 8.0 Hz, 1H), 2.55 (s, 3H); FAB mass spectrum, 351.0172 (M+H calcd for C₁₆H₁₅O₃S₃, 351.0184).

### Compound 32

Slightly yellow prisms from CH₂Cl₂: m.p. >130° d; IR (CHCl₃) 3610-3150, 3570, 3010, 1665, 1125, 1045 cm⁻¹; ¹H NMR (250 MHz, CDCl₃) δ 7.35-7.27 (m, 5H), 6.44-6.37 (m, 1H), 6.09 (s, 1H), 5.91 (d, J = 9.6 Hz, 1H), 5.85 (dd, J = 1.4, 9.5 Hz, 1H), 5.49 (d, J = 8.0 Hz, 1H), 4.08 (app s, 2H), 3.89 (1/2 ABX, J_{AB} = 13.5, J_{AX} = 8.8 Hz, 1H), 3.79 (1/2 ABX, J_{BA}= 13.5, J_{BX} = 7.5 Hz, 1H), 3.08 (dd, J = 1.0, 17.1 Hz, 1H), 2.71 (d, J = 17.1 Hz, 1H), 2.62 (s, 1H), 2.58 (d, J = 8.1 Hz, 1H); FAB mass spectrum, 427.0478 (M+H calcd for C₂₂H₁₉O₃S₃, 427.0556.

### Compound 33

Colorless prisms from THF/hexanes: m.p. 207-209°C; IR (KBr) 3590-3100, 2880, 1700, 1235, 1075, 1035 cm⁻¹; ¹H NMR (250 MHz, CDCl₃) δ 7.60 (d, J = 8.4 Hz, 1H), 7.45-7.30 (m, 3H), 6.03 (app t, J = 2.4 Hz, 1H), 4.43 (d, J = 0.5 Hz, 1H), 3.93 (app t, J = 1.8 Hz, 2H), 3.59 (d, J = 1.2 Hz, 1H), 3.20 (d, J = 16.6 Hz, 1H), 2.88 (d, J = 13.6 Hz, 1H), 2.87 (dd, J = 1.8, 16.6 Hz, 1H), 2.73 (dd, J = 1.9, 14.0 Hz, 1H), 2.63 (s, 1H); electron impact mass spectrum, 274.0659 (M+ calcd for C₁₅H₁₄O₃S, 274.0664).

### Compound 35

A colorless glass: IR (CHCl₃) 3610-3210, 3415, 2930, 1718, 1499, 1084 cm⁻¹; ¹H NMR (250 MHz, CDCl₃) δ 7.61 (d, J = 7.9 Hz, 1H), 7.46 - 7.39 (m, 1H), 7.32 (app d, J = 4.1 Hz, 2H), 6.16 (app t, J = 2.4 Hz, 1H), 5.19 (s, 2H), 4.54 (s, 1H), 3.95 (app t, J = 3.5 Hz, 2H), 3.73 (s, 3H), 3.44 (d, J = 0.7 Hz, 1H), 2.90 (ABq, J = 12.5, Δv = 52.1 Hz, 2H), 2.64 (s, 1H).

Although the present invention has now been described in terms of certain preferred embodiments, and exemplified with respect thereto, one skilled in the art will readily appreciate that various modifications, changes, omissions and substitutions may be made within the scope of the attached claims.

## Claims

1. A compound havlng the structural formula 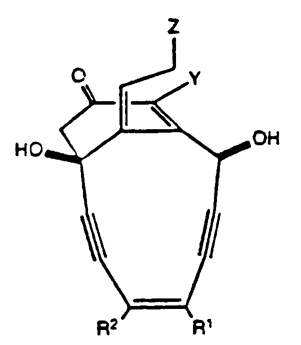 wherein R¹ and R² are the same or different and are selected from the group consisting of hydrogen, C₁-C₆ alkyl, phenyl, C₁-C₆ alkyl-substituted phenyl, benzyl and C₁-C₆ alkyl-substituted benzyl, or
R¹ and R² together with the intervening vinylene group form a phenyl group or a C₁-C₆ alkyl-substituted phenyl group;
Y is selected from the group consisting of hydrogen and NHR³ wherein R³ is selected from the group consisting of C₁-C₆ alkyl, C₁-C₆ alkanoyl, benzoyl, and carbonyl C₁-C₆ alkoxy; and
Z is selected from the group consisting of hydroxyl, C₁-C₆ acyloxy, C₁-C₆ acylthioxy, benzoyloxy, benzoylthioxy, and SSSR⁴ where R⁴ is C₁-C₆ alkyl or benzyl, with the proviso that Z is other than hydroxyl or C₁-C₆ acylthioxy when Y is hydrogen, or R² and R¹ are both hydrogen.

2. The compound of claim 1 wherein R¹ and R² are both hydrogen.

3. The compound of claim 2 wherein Y is hydrogen.

4. The compound of claim 2 wherein Y is NHR³ and R³ is carbonyl C₁- C₆ alkoxy.

5. The compound of claim 2 wherein Z is SSSR⁴ and R⁴ is methyl or benzyl.

6. Calicheamicinone.

7. A compound having the structural formula 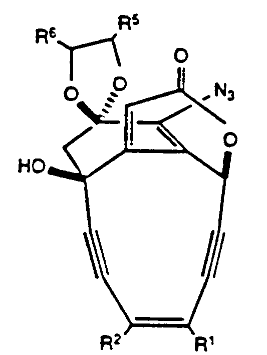 wherein R¹ and R² are the same or different and are selected from the group consisting of hydrogen, C₁-C₆ alkyl, phenyl, C₁-C₆ alkyl-substituted phenyl, benzyl and C₁-C₆ alkyl-substituted benzyl, or
R¹ and R² together with the intervening vinylene group form a phenyl group or a C₁-C₆ alkyl- substituted phenyl group; and
R⁵ and R⁶ are the same or different and are hydrogen or C₁-C₃ alkyl, or R⁵ and R⁶ together with the intervening ethylene group comprise a 5- or 6-membered ring.

8. The compound of claim 7 wherein R¹, R², R⁵ and R⁶ are hydrogen.

9. A compound having the structural formula 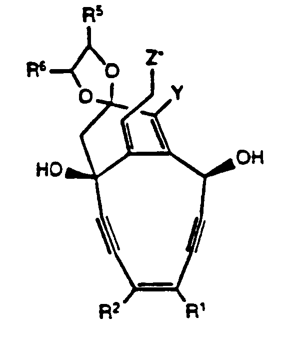 wherein R¹ and R² are the same or different and are selected from the group consisting of hydrogen, C₁-C₆ alkyl, phenyl, C₁-C₆ alkyl-substituted phenyl, benzyl and C₁-C₆ alkyl-substituted benzyl, or
R¹ and R² together with the intervening vinylene group form a phenyl group or a C₁-C₆ alkyl-substituted phenyl group;
R⁵ and R⁶ are the same or different and are hydrogen or C₁-C₃ alkyl, or R⁵ and R⁶ together with the intervening ethylene group comprise a 5- or 6-membered ring;
Y is selected from the group consisting of hydrogen and NHR³ wherein R³ is selected from the group consisting of C₁-C₆ alkyl, C₁-C₆ alkanoyl, benzoyl, and carbonyl C₁-C₆ alkoxy; and
Z* is selected from the group consisting of hydroxyl, mercaptan, C₁-C₆ acyloxy, C₁-C₆ acylthioxy, benzoyloxy, benzoylthioxy, and SSSR⁴ where R⁴ is C₁-C₆ alkyl or benzyl, with the proviso that Z* is other than hydroxyl or C₁-C₆ acylthioxy when Y is hydrogen or R² and R¹ are both hydrogen.

10. The compound of claim 9 wherein R¹, R², R⁵ and R⁶ are hydrogen.

11. The compound of claim 10 wherein Y is hydrogen.

12. The compound of claim 11 wherein Z* is SSSR⁴.

13. A method of preparing a compound having the structure of Formula VI 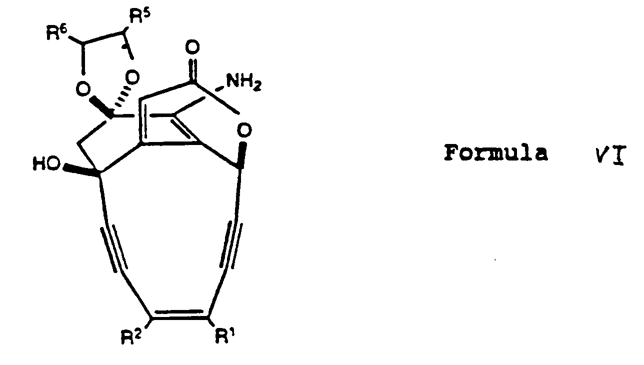 wherein R¹ and R² are the same or different and are selected from the group consisting of hydrogen, C₁-C₆ alkyl, phenyl, C₁-C₆ alkyl-substituted phenyl, benzyl and C₁-C₆ alkyl-substituted benzyl, or
R¹ and R² together with the intervening vinylene group form a phenyl group or a C₁-C₆ alkyl-substituted phenyl group;
R⁵ and R⁶ are the same or different and are hydrogen or C₁-C₃ alkyl, or R⁵ and R⁶ together with the intervening ethylene group comprise a 5- or 6-membered ring;
that comprises the steps of
(a) reacting a compound having the structure of Formula VII 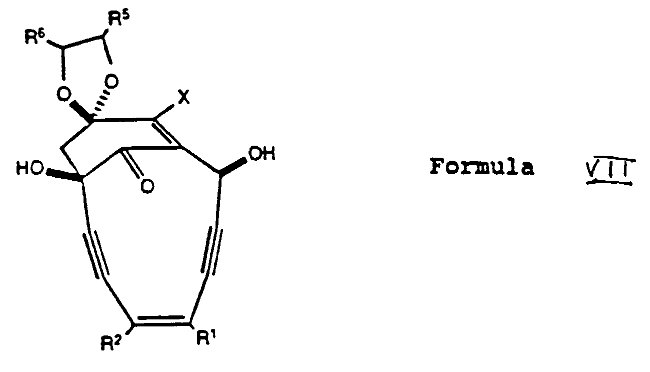 wherein R¹, R², R⁵ and R⁶ are defined above, and X is a halo group to
(i) replace the halo substituent with an azido substituent;
(ii) esterify the allylic hydroxyl group to form an ester whose carboxyl portion has the formula -C(O)CH₂P(O)(OR⁷)₂ wherein R⁷ is C₁-C₆ lower alkyl; and
(iii) carry out an intramolecular Emmons condensation to form a compound having the structure of Formula VIII 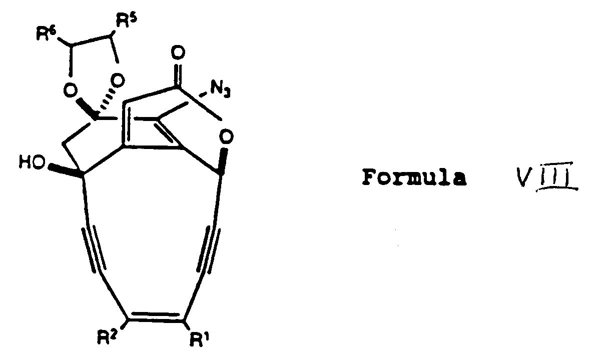 whereln R¹, R², R⁵ and R⁶ are as defined above;
(b) recovering the compound of step (a); and
(c) reducing the recovered compound of step (b) to form the compound of Formula VI.

14. The method of claim 13 wherein R¹, R², R⁵ and R⁶ are hydrogen.

15. The method of claim 13 including the further step of recovering the compound of Formula VI formed in step (c).

16. A pharmaceutical composition comprising a physiologically acceptable diluent having dissolved or dispersed therein an effective amount of a compound having the structural formula 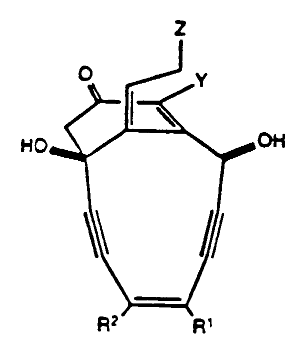 wherein R¹ and R² are the same or different and are selected from the group consisting of hydrogen, C₁-C₆ alkyl, phenyl, C₁-C₆ alkyl-substituted phenyl, benzyl and C₁-C₆ alkyl-substituted benzyl, or
R¹ and R² together with the intervening vinylene group form a phenyl group or a C₁-C₆ alkyl-substituted phenyl group;
Y is selected from the group consisting of hydrogen and NHR³ whereln R³ is selected from the group consisting of C₁-C₆ alkyl, C₁-C₆ alkanoyl, benzoyl, and carbonyl C₁-C₆ alkoxy; and
Z is selected from the group consisting of hydroxyl, C₁-C₆ acyloxy, C₁-C₆ acylthioxy, benzoyloxy, benzoylthioxy, and SSSR⁴ where R⁴ is C₁-C₆ alkyl or benzyl, with the proviso that Z is other than hydroxyl or C₁-C₆ acylthioxy when Y is hydrogen, or R² and R¹ are both hydrogen.

17. The pharmaceutical composition of claim 16 wherein R¹ and R² are both hydrogen.

18. The pharmaceutical composition of claim 17 wherein Y is hydrogen.

19. The pharmaceutical composition of claim 17 wherein Y is NHC(O)OCH₃ and Z is SSSR⁴.

## Patentansprüche

1. Verbindung mit der Strukturformel 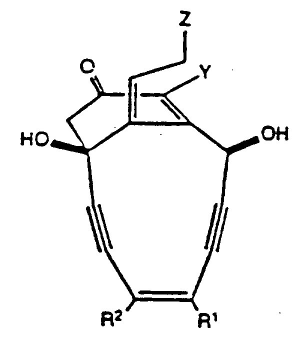 worin R¹ und R² gleich oder verschieden sind und ausgewählt sind aus der Gruppe von Wasserstoff, C₁-C₆-Alkyl, Phenyl, C₁-C₆-Alkyl-substituiertem Phenyl, Benzyl und C₁-C₆-Alkyl-substituiertem Benzyl, oder
R¹ und R² zusammen mit der dazwischenliegenden Vinylengruppe eine Phenylgruppe oder eine C₁-C₆-Alkyl-substituierte Phenylgruppe bilden;
Y ausgewählt ist aus der Gruppe von Wasserstoff und NHR³, worin R³ ausgewählt ist aus der Gruppe von C₁-C₆-Alkyl, C₁-C₆-Alkanoyl, Benzoyl und Carbonyl-C₁-C₆-alkoxy; und
Z ausgewählt ist aus der Gruppe von Hydroxyl, C₁-C₆-Acyloxy, C₁-C₆-Acylthioxy, Benzoyloxy, Benzoylthioxy und SSSR⁴, worin R⁴ ein C₁-C₆-Alkyl oder Benzyl ist, mit der Maßgabe, daß Z nicht Hydroxyl oder C₁-C₆-Acylthioxy ist, wenn Y Wasserstoff ist oder R² und R¹ beide Wasserstoff sind.

2. Verbindung nach Anspruch 1, worin R¹ und R² beide Wasserstoff sind.

3. Verbindunng nach Anspruch 2, worin Y Wasserstoff ist.

4. Verbindung nach Anspruch 2, worin Y NHR³ ist und R³ Carbonyl-C₁-C₆-alkoxy ist.

5. Verbindung nach Anspruch 2, worin Z SSSR⁴ ist und R⁴ Methyl oder Benzyl ist.

6. Calicheamicinon.

7. Verbindung mit der Strukturformel 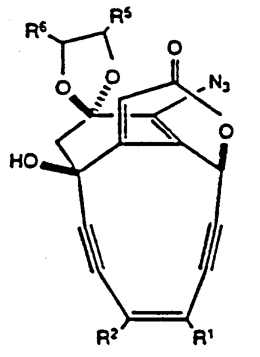 worin R¹ und R² gleich oder verschieden Sind und ausgewählt sind aus der Gruppe von Wasserstoff, C₁-C₆-Alkyl, Phenyl, C₁-C₆-Alkyl-substituiertem Phenyl, Benzyl und C₁-C₆-Alkyl-substituiertem Benzyl, oder
R¹ und R² zusammen mit der dazwischenliegenden Vinylengruppe eine Phenylgruppe oder eine C₁-C₆-Alkyl-substituierte Phenylgruppe bilden; und
R⁵ und R⁶ gleich oder verschieden sind und Wasserstoff oder C₁-C₃-Alkyl sind, oder R⁵ und R⁶ zusammen mit der dazwischenliegenden Ethylengruppe einen 5- oder 6-gliedrigen Ring umfassen.

8. Verbindung nach Anspruch 7, worin R¹, R², R⁵ und R⁶ Wasserstoff sind.

9. Verbindung mit der Strukturformel 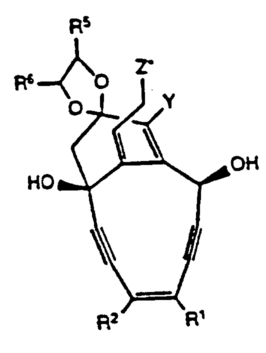 worin R¹ und R² gleich oder verschieden sind und ausgewählt sind aus der Gruppe von Wasserstoff, C₁-C₆-Alkyl, Phenyl, C₁-C₆-Alkyl-substituiertem Phenyl, Benzyl und C₁-C₆-Alkyl-substituiertem Benzyl, oder
R¹ und R² zusammen mit der dazwischenliegenden Vinylengruppe eine Phenylgruppe oder eine C₁-C₆-Alkyl-substituierte Phenylgruppe bilden; und
R⁵ und R⁶ gleich oder verschieden sind und Wasserstoff oder C₁-C₃-Alkyl sind, oder R⁵ und R⁶ zusammen mit der dazwischenliegenden Ethylengruppe einen 5- oder 6-gliedrigen Ring umfassen;
Y ausgewählt ist aus der Gruppe von Wasserstoff und NHR³, worin R³ ausgewählt ist aus der Gruppe von C₁-C₆-Alkyl, C₁-C₆-Alkanoyl, Benzoyl und Carbonyl-C₁-C₆-alkoxy; und
Z* ausgewählt ist aus der Gruppe von Hydroxyl, Mercaptan, C₁-C₆-Acyloxy, C₁-C₆-Acylthioxy, Benzoyloxy, Benzoylthioxy und SSSR⁴, worin R⁴ ein C₁-C₆-Alkyl oder Benzyl ist, mit der Maßgabe, daß Z* nicht Hydroxyl oder C₁-C₆-Acylthioxy ist, wenn Y Wasserstoff ist oder R² und R¹ beide Wasserstoff sind.

10. Verbindung nach Anspruch 9, worin R¹, R², R⁵ und R⁶ Wasserstoff sind.

11. Verbindung nach Anspruch 10, worin Y Wasserstoff ist.

12. Verbindung nach Anspruch 11, worin Z* SSSR⁴ ist.

13. Verfahren zum Herstellen einer Verbindung mit der Struktur von Formel VI 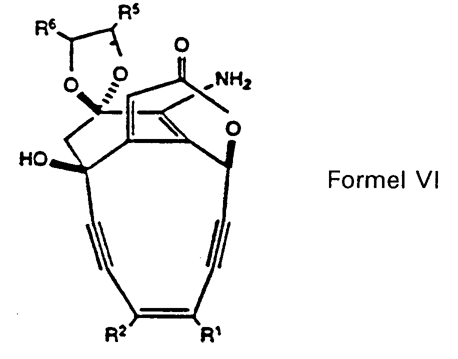 worin R¹ und R² gleich oder verschieden sind und ausgewählt sind aus der Gruppe von Wasserstoff, C₁-C₆-Alkyl, Phenyl, C₁-C₆-Alkyl-substituiertem Phenyl, Benzyl und C₁-C₆-Alkyl-substituiertem Benzyl, oder
R¹ und R² zusammen mit der dazwischenliegenden Vinylengruppe eine Phenylgruppe oder eine C₁-C₆-Alkyl-substituierte Phenylgruppe bilden;
R⁵ und R⁶ gleich oder verschieden sind und Wasserstoff oder C₁-C₃-Alkyl sind, oder R⁵ und R⁶ zusammen mit der dazwischenliegenden Ethylengruppe einen 5- oder 6-gliedrigen Ring umfassen;
welches die folgenden Schritte umfaßt
(a) Umsetzen einer Verbindung mit der Struktur von Formel VII 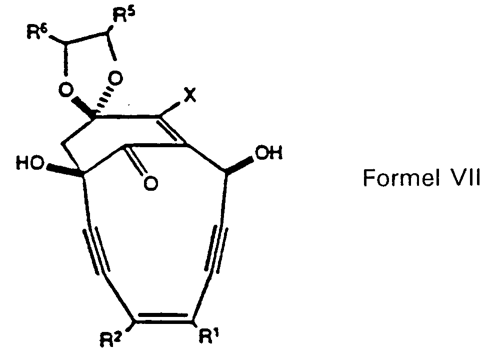 worin R¹, R², R⁵ und R⁶ wie vorstehend definiert sind, und X eine Halogengruppe ist, um
(i) den Halogen-Substituenten gegen einen Azido-Substituenten auszutauschen;
(ii) die allylische Hydroxylgruppe unter Bildung eines Esters, dessen Carboxylteil die Formel -C(O)CH₂P(O)(OR⁷)₂ aufweist, worin R⁷ C₁-C₆-Niedrigalkyl ist, zu verestern; und
(iii) eine intramolekulare Emmons-Kondensation unter Bildung einer Verbindung mit der Struktur von Formel VIII durchzuführen 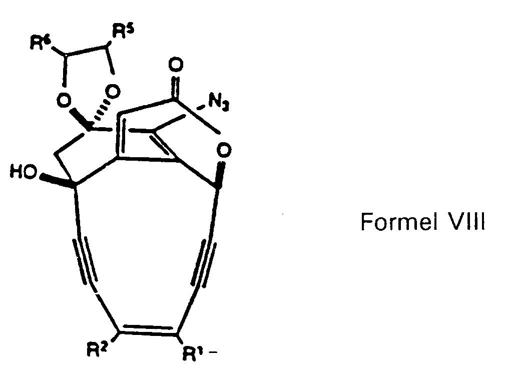 worin R¹, R², R⁵ und R⁶ wie vorstehend definiert sind;
(b) Gewinnen der Verbindung von Schritt (a);
(c) Reduzieren der gewonnenen Verbindung von Schritt (b) unter Bildung der Verbindung mit Formel VI.

14. Verfahren nach Anspruch 13, worin R¹, R², R⁵ und R⁶ Wasserstoff sind.

15. Verfahren nach Anspruch 13, das als weiteren Schritt das Gewinnen der in Schritt (c) gebildeten Verbindung mit der Formel VI einschließt.

16. Pharmazeutische Zusammensetzung umfassend ein physiologisch verträgliches Verdünnungsmittel, das eine wirksame Menge einer Verbindung gelöst oder dispergiert enthält, wobei die Verbindung die folgende Strukturformel aufweist 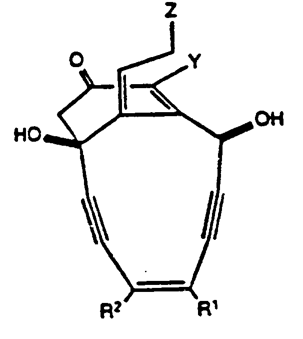 worin R¹ und R² gleich oder verschieden sind und ausgewählt sind aus der Gruppe von Wasserstoff, C₁-C₆-Alkyl, Phenyl, C₁-C₆-Alkyl-substituiertem Phenyl, Benzyl und C₁-C₆-Alkyl-substituiertem Benzyl, oder
R¹ und R² zusammen mit der dazwischenliegenden Vinylengruppe eine Phenylgruppe oder eine C₁-C₆-Alkyl-substituierte Phenylgruppe bilden;
Y ausgewählt ist aus der Gruppe von Wasserstoff und NHR³, worin R³ ausgewählt ist aus der Gruppe von C₁-C₆-Alkyl, C₁-C₆-Alkanoyl, Benzoyl und Carbonyl-C₁-C₆-alkoxy;
Z ausgewählt ist aus der Gruppe von Hydroxyl, C₁-C₆-Acyloxy, C₁-C₆-Acylthioxy, Benzoyloxy, Benzoylthioxy und SSSR⁴, worin R⁴ ein C₁-C₆-Alkyl oder Benzyl ist, mit der Maßgabe, daß Z nicht Hydroxyl oder C₁-C₆-Acylthioxy ist, wenn Y Wasserstoff ist oder R² und R¹ beide Wasserstoff sind.

17. Pharmazeutische Zusammensetzung nach Anspruch 16, worin R¹ und R² beide Wasserstoff sind.

18. Pharmazeutische Zusammensetzung nach Anspruch 17, worin Y Wasserstoff ist.

19. Pharmazeutische Zusammensetzung nach Anspruch 17, worin Y NHC(O)OCH₃ ist und Z SSSR⁴ ist.

## Revendications

1. Composé ayant la formule développée: 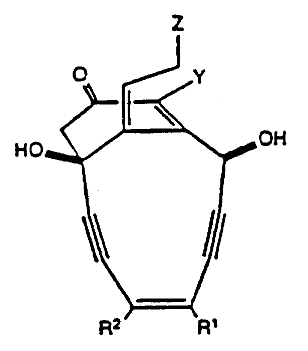 dans laquelle R¹ et R² sont identiques ou différents et sont choisis dans le groupe formé par l'atome d'hydrogène, les radicaux alkyle en C₁-C₆, phényle, phényle substitué par un groupe alkyle en C₁-C₆, benzyle et benzyle substitué par un groupe alkyle en C₁- C₆, ou bien
R¹ et R² conjointement avec le groupe vinylène intermédiaire forment un groupe phényle ou un groupe phényle substitué par un groupe alkyle en C₁-C₆;
Y est choisi dans le groupe formé par l'atome d'hydrogène et le groupe NHR³ dans lequel R³ est choisi dans le groupe formé par les radicaux alkyle en C₁-C₆, alcanoyle en C₁-C₆, benzoyle et carbonylalcoxy en C₁-C₆; et
Z est choisi dans le groupe formé par les radicaux hydroxyle, acyloxy en C₁-C₆, acylthioxy en C₁-C₆, benzoyloxy, benzoylthioxy et SSSR⁴ où R⁶ est un groupe alkyle en C₁-C₆ ou benzyle, à condition que Z soit autre que le groupe hydroxyle ou acylthioxy en C₁-C₆ quand Y est un atome d'hydrogène, ou bien R² et R¹ sont tous deux un atome d'hydrogène.

2. Composé selon la revendication 1, dans lequel R¹ et R² sont tous deux un atome d'hydrogène.

3. Composé selon la revendication 2, dans lequel Y est un atome d'hydrogène.

4. Composé selon la revendication 2, dans lequel Y est NHR³, et R³ est un groupe carbonylalcoxy en C₁-C₆.

5. Composé selon la revendication 2, dans lequel Z est SSSR⁴ et R⁴ est un groupe méthyle ou benzyle.

6. Calicheamicinone.

7. Composé ayant la formule développée 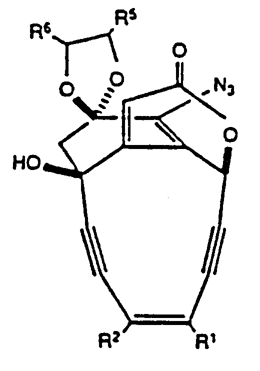 dans laquelle R¹ et R² sont identiques ou différents et sont choisis dans le groupe formé par l'atome d'hydrogène, les radicaux alkyle en C₁-C₆, phényle, phényle substitué par un groupe alkyle en C₁-C₆, benzyle et benzyle substitué par un groupe alkyle en C₁-C₆, ou bien
R¹ et R² conjointement avec le groupe vinylène intermédiaire forment un groupe phényle ou un groupe phényle substitué par un groupe alkyle en C₁-C₆; et
R⁵ et R⁶ sont identiques ou différents et sont un atome d'hydrogène ou un groupe alkyle en C₁-C₃, ou bien R⁵ et R⁶ conjointement avec le groupe éthylène intermédiaire forment un cycle à 5 ou 6 chaînons.

8. Composé selon la revendication 7, dans lequel R¹ R², R⁵ et R⁶ sont un atome d'hydrogène.

9. Composé ayant la formule développée 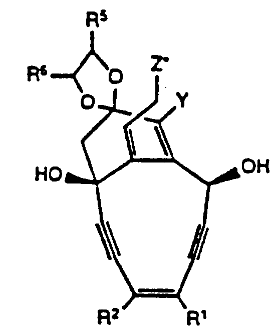 dans laquelle R¹ et R² sont identiques ou différents et sont choisis dans le groupe formé par l'atome d'hydrogène, les radicaux alkyle en C₁-C₆, phényle, phényle substitué par un groupe alkyle en C₁-C₆, benzyle et benzyle substitué par un groupe alkyle en C₁-C₆, ou bien
R¹ et R² conjointement avec le groupe vinylène intermédiaire forment un groupe phényle ou un groupe phényle substitué par un groupe alkyle en C₁-C₆;
R⁵ et R⁶ sont identiques ou différents et représentent un atome d'hydrogène ou un groupe alkyle en C₁-C₃, ou bien R⁵ et R⁶ conjointement avec le groupe éthylène intermédiaire forment un cycle à 5 ou 6 chaînons;
Y est choisi dans le groupe formé par l'atome d'hydrogène et le groupe NHR³ dans lequel R³ est choisi dans le groupe formé par les radicaux alkyle en C₁-C₆, alcanoyle en C₁-C₆, benzoyle, et carbonylalcoxy en C₁-C₆; et
Z* est choisi dans le groupe formé par les radicaux hydroxyle, mercaptan, acyloxy en C₁-C₆, acylthioxy en C₁-C₆, benzoyloxy, benzoylthioxy et SSSR⁴ où R⁴ est un groupe alkyle en C₁-C₆ ou benzyle, à condition que Z* soit autre que le groupe hydroxyle ou acylthioxy en C₁-C₆ quand Y est un atome d'hydrogène ou bien R² et R¹ sont tous deux un atome d'hydrogène.

10. Composé selon la revendication 9, dans lequel R¹, R², R⁵ et R⁶ sont un atome d'hydrogène.

11. Composé selon la revendication 10, dans lequel Y est un atome d'hydrogène.

12. Composé selon la revendication 11, dans lequel Z* est SSSR⁴.

13. Procédé pour préparer un composé ayant la structure de formule VI 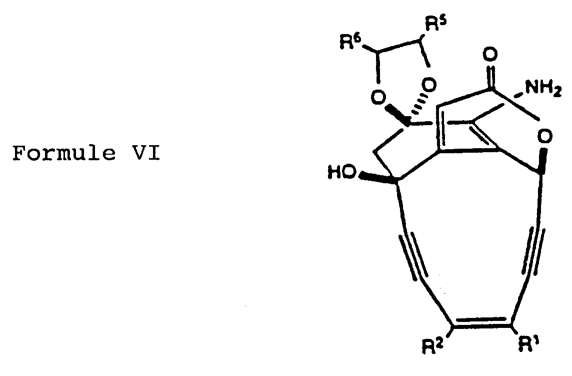 dans laquelle R¹ et R² sont identiques ou différents et sont choisis dans le groupe formé par l'atome d'hydrogène, les radicaux alkyle en C₁-C₆, phényle, phényle substitué par un groupe alkyle en C₁-C₆, benzyle et benzyle substitué par un groupe alkyle en C₁-C₆, ou bien
R¹ et R² conjointement avec le groupe vinylène intermédiaire forment un groupe phényle ou un groupe phényle substitué par un groupe alkyle en C₁-C₆;
R⁵ et R⁶ sont identiques ou différents et sont un atome d'hydrogène ou un groupe alkyle en C₁-C₃, ou bien R⁵ et R⁶ conjointement avec le groupe éthylène intermédiaire forment un cycle à 5 ou 6 chaînons;
qui comprend les étapes consistant:
(a) à faire réagir un composé ayant la structure de formule VII 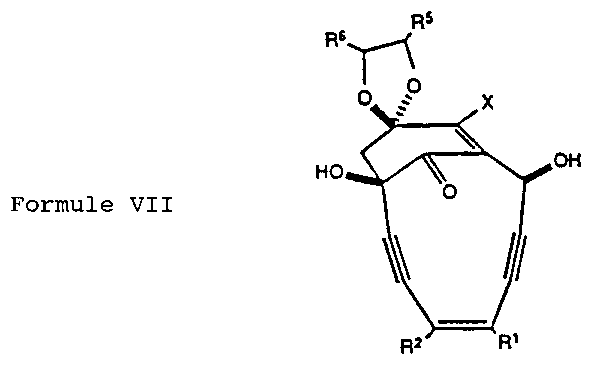 dans laquelle R¹, R², R⁵ et R⁶ sont comme définis ci-dessus, et X est un groupe halogéno pour
(i) remplacer le substituant halogéno par un substituant azido;
(ii) estérifier le groupe hydroxyle allylique pour former un ester dont la partie carboxyle possède la formule -C(O)CH₂P(O)(OR⁷)₂ dans laquelle R⁷ est un groupe alkyle inférieur en C₁-C₆; et
(iii) effectuer une condensation intramoléculaire d'Emmons pour former un composé ayant la structure de formule VIII 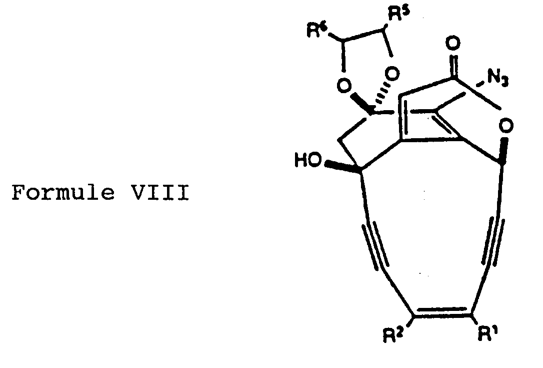 dans laquelle R¹, R², R⁵ et R⁶ sont tels que définis ci-dessus;
(b) à récupérer le composé de l'étape (a); et
(c) à réduire le composé récupéré de l'étape (b) pour former le composé de formule VI.

14. Procédé selon la revendication 13, dans lequel R¹, R², R⁵ et R⁶ représentent un atome d'hydrogène.

15. Procédé selon la revendication 13, incluant l'étape supplémentaire consistant à récupérer le composé de formule VI formé dans l'étape (c).

16. Composition pharmaceutique comprenant un diluant acceptable sur le plan physiologique y ayant dissous ou dispersé une quantité efficace d'un composé ayant la formule développée 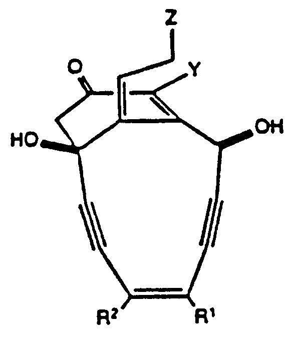 dans laquelle R¹ et R² sont identiques ou differénts et sont choisis dans le groupe formé par l'atome d'hydrogène, les radicaux alkyle en C₁-C₆, phényle, phényle substitué par un groupe alkyle en C₁-C₆, benzyle et benzyle substitué par un groupe alkyle en C₁-C₆, ou bien
R¹ et R² conjointement avec le groupe vinylène intermédiaire forment un groupe phényle ou un groupe phényle substitué par un groupe alkyle en C₁-C₆;
Y est choisi dans le groupe formé par l'atome d'hydrogène et le groupe NHR³ dans lequel R³ est choisi dans le groupe formé par les radicaux alkyle en C₁-C₆, alcanoyle en C₁-C₆, benzoyle, et carbonylalcoxy en C₁-C₆; et
Z est choisi dans le groupe formé par les radicaux hydroxyle, acyloxy en C₁-C₆, acylthioxy en C₁-C₆, benzoyloxy, benzoylthioxy et SSSR⁴ où R⁴ est un groupe alkyle en C₁-C₆ ou benzyle, à condition que Z soit autre que le groupe hydroxyle ou acylthioxy en C₁-C₆ quand Y représente un atome d'hydrogène, ou bien R² et R¹ sont tous deux un atome d'hydrogène.

17. Composition pharmaceutique selon la revendication 16, dans laquelle R¹ et R² sont tous deux un atome d'hydrogène.

18. Composition pharmaceutique selon la revendication 17, dans laquelle Y est un atome d'hydrogène.

19. Composition pharmaceutique selon la revendication 17, dans laquelle Y est NHC(O)OCH₃ et Z est SSSR⁴.
